(19) Europäisches Patentamt
European Patent Office
Office européen des brevets

(11) EP 3 153 178 A1

(12) # EUROPEAN PATENT APPLICATION
published in accordance with Art. 153(4) EPC

(43) Date of publication:
**12.04.2017 Bulletin 2017/15**

(21) Application number: **15803346.4**

(22) Date of filing: **05.06.2015**

(51) Int Cl.:
***A61K 45/00*** *(2006.01)* ***A61K 31/519*** *(2006.01)*
***A61K 39/395*** *(2006.01)* ***A61P 35/00*** *(2006.01)*

(86) International application number:
**PCT/JP2015/066328**

(87) International publication number:
**WO 2015/186823 (10.12.2015 Gazette 2015/49)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(30) Priority: **06.06.2014 US 201462008726 P**

(71) Applicant: **Kyowa Hakko Kirin Co., Ltd.**
**Tokyo 100-8185 (JP)**

(72) Inventors:
- **ANDO Munetoshi**
  **Tokyo 100-8185 (JP)**
- **GOTO Keiko**
  **Tokyo 100-8185 (JP)**

(74) Representative: **Tuxworth, Pamela M.**
**J A Kemp**
**14 South Square**
**Gray's Inn**
**London WC1R 5JJ (GB)**

(54) **METHOD FOR TREATING CANCER PATIENTS USING FOLR1 TARGETED DRUG AND ANTIFOLATE, AND DRUG**

(57) The invention provides a method for treating a cancer with a human FOLR1-targeting drug by increasing an expression of folate receptor α (hereinafter abbreviated to FOLR1) by using an antagonist for folic acid metabolism, a medicament for a cancer containing a human FOLR1-targeting drug for increasing an expression of FOLR1 by using an antagonist for folic acid metabolism and a method for enhancing a therapeutic effect of a human FOLR1-targeting drug by increasing an expression of FOLR1 in a cancer cell by using an antagonist for folic acid metabolism. For example, the invention provides a more effective therapeutic method using an antagonist for folic acid metabolism with a human FOLR1-targeting drug, for cancer patients in which an expression level of FOLR1 is low and in which an anti-tumor activity of the human FOLR1-targeting drug is not exhibited sufficiently or for cancer patients in which a treatment of a cancer expressing FOLR1 is to be further enhanced.

EP 3 153 178 A1

SEQUENCE LISTING

<110> Kyowa Hakko Kirin Co., Ltd.

<120> Therapeutic method and medicament for cancer patients by FOLR1-targeting drug and an antagonist for folic acid metabolism

<130> W518541

<150> US62/008726
<151> 2014-06-06

<160> 13

<170> PatentIn version 3.3

<210> 1
<211> 257
<212> PRT
<213> Homo sapiens

<400> 1

```
Met Ala Gln Arg Met Thr Thr Gln Leu Leu Leu Leu Leu Val Trp Val
1               5                   10                  15

Ala Val Val Gly Glu Ala Gln Thr Arg Ile Ala Trp Ala Arg Thr Glu
            20                  25                  30

Leu Leu Asn Val Cys Met Asn Ala Lys His His Lys Glu Lys Pro Gly
        35                  40                  45

Pro Glu Asp Lys Leu His Glu Gln Cys Arg Pro Trp Arg Lys Asn Ala
    50                  55                  60

Cys Cys Ser Thr Asn Thr Ser Gln Glu Ala His Lys Asp Val Ser Tyr
65                  70                  75                  80

Leu Tyr Arg Phe Asn Trp Asn His Cys Gly Glu Met Ala Pro Ala Cys
                85                  90                  95

Lys Arg His Phe Ile Gln Asp Thr Cys Leu Tyr Glu Cys Ser Pro Asn
            100                 105                 110

Leu Gly Pro Trp Ile Gln Gln Val Asp Gln Ser Trp Arg Lys Glu Arg
        115                 120                 125

Val Leu Asn Val Pro Leu Cys Lys Glu Asp Cys Glu Gln Trp Trp Glu
    130                 135                 140

Asp Cys Arg Thr Ser Tyr Thr Cys Lys Ser Asn Trp His Lys Gly Trp
145                 150                 155                 160
```

```
Asn Trp Thr Ser Gly Phe Asn Lys Cys Ala Val Gly Ala Ala Cys Gln
                165                 170                 175

Pro Phe His Phe Tyr Phe Pro Thr Pro Thr Val Leu Cys Asn Glu Ile
            180                 185                 190

Trp Thr His Ser Tyr Lys Val Ser Asn Tyr Ser Arg Gly Ser Gly Arg
        195                 200                 205

Cys Ile Gln Met Trp Phe Asp Pro Ala Gln Gly Asn Pro Asn Glu Glu
    210                 215                 220

Val Ala Arg Phe Tyr Ala Ala Ala Met Ser Gly Ala Gly Pro Trp Ala
225                 230                 235                 240

Ala Trp Pro Phe Leu Leu Ser Leu Ala Leu Met Leu Leu Trp Leu Leu
                245                 250                 255

Ser


<210>  2
<211>  5
<212>  PRT
<213>  Artificial

<220>
<223>  RA15-7VHCDR1_AA

<400>  2

Asp Phe Tyr Met Asn
1               5


<210>  3
<211>  19
<212>  PRT
<213>  Artificial

<220>
<223>  RA15-7VHCDR2_AA

<400>  3

Phe Ile Arg Asn Lys Ala Asn Gly Tyr Thr Thr Glu Phe Asn Pro Ser
1               5                   10                  15

Val Lys Gly


<210>  4
<211>  13
<212>  PRT
```

<213>  Artificial

<220>
<223>  RA15-7VHCDR3_AA

<400>  4

Cys Leu Tyr Gly Tyr Ala Tyr Tyr Tyr Val Met Asp Ala
1               5                   10

<210>  5
<211>  11
<212>  PRT
<213>  Artificial

<220>
<223>  RA15-7VLCDR1_AA

<400>  5

Arg Thr Ser Glu Asp Ile Phe Arg Asn Leu Ala
1               5                   10

<210>  6
<211>  7
<212>  PRT
<213>  Artificial

<220>
<223>  RA15-7VLCDR2_AA

<400>  6

Asp Thr Asn Arg Leu Ala Asp
1               5

<210>  7
<211>  9
<212>  PRT
<213>  Artificial

<220>
<223>  RA15-7VLCDR3_AA

<400>  7

Gln Gln Tyr Asp Asn Tyr Pro Leu Thr
1               5

<210>  8
<211>  124
<212>  PRT
<213>  Artificial

<220>
<223>  nonS_HuRA15-7CTVH_AA

<400>  8

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Thr Asp Phe
            20                  25                  30

Tyr Met Asn Trp Val Arg Gln Pro Pro Gly Lys Ala Pro Glu Trp Leu
        35                  40                  45

Gly Phe Ile Arg Asn Lys Ala Asn Gly Tyr Thr Thr Glu Phe Asn Pro
    50                  55                  60

Ser Val Lys Gly Arg Phe Thr Ile Ser Arg Asp Asn Ser Lys Asn Ser
65                  70                  75                  80

Leu Tyr Leu Gln Met Asn Ser Leu Lys Thr Glu Asp Thr Ala Thr Tyr
                85                  90                  95

Tyr Cys Ala Arg Thr Leu Tyr Gly Tyr Ala Tyr Tyr Tyr Val Met Asp
            100                 105                 110

Ala Trp Gly Gln Gly Thr Leu Val Thr Val Ser Ser
        115                 120

<210> 9
<211> 107
<212> PRT
<213> Artificial

<220>
<223> nonS_HuRA15-7LV3_AA

<400> 9

Asp Ile Gln Met Thr Gln Ser Pro Ser Ser Leu Ser Ala Ser Leu Gly
1               5                   10                  15

Asp Arg Val Thr Ile Thr Cys Arg Thr Ser Glu Asp Ile Phe Arg Asn
            20                  25                  30

Leu Ala Trp Tyr Gln Gln Lys Pro Gly Lys Ala Pro Lys Leu Leu Ile
        35                  40                  45

Tyr Asp Thr Asn Arg Leu Ala Asp Gly Val Pro Ser Arg Phe Ser Gly
    50                  55                  60

Ser Gly Ser Gly Thr Asp Tyr Thr Leu Thr Ile Ser Ser Leu Gln Pro
65                  70                  75                  80

Glu Asp Phe Ala Thr Tyr Phe Cys Gln Gln Tyr Asp Asn Tyr Pro Leu

85 90 95

Thr Phe Gly Gln Gly Thr Lys Leu Glu Ile Lys
100 105

<210> 10
<211> 124
<212> PRT
<213> Artificial

<220>
<223> nonS_RA15-7VH_AA

<400> 10

Glu Val Lys Leu Leu Glu Ser Gly Gly Gly Leu Val Gln Pro Gly Gly
1 5 10 15

Ser Met Arg Leu Ser Cys Ala Ala Ser Gly Phe Thr Phe Thr Asp Phe
20 25 30

Tyr Met Asn Trp Ile Arg Gln Pro Ala Gly Lys Ala Pro Glu Trp Leu
35 40 45

Gly Phe Ile Arg Asn Lys Ala Asn Gly Tyr Thr Thr Glu Phe Asn Pro
50 55 60

Ser Val Lys Gly Arg Phe Ala Ile Ser Arg Asp Asn Thr Gln Asn Met
65 70 75 80

Leu Tyr Leu Gln Met Asn Thr Leu Arg Ala Glu Asp Thr Ala Thr Tyr
85 90 95

Tyr Cys Ala Arg Cys Leu Tyr Gly Tyr Ala Tyr Tyr Tyr Val Met Asp
100 105 110

Ala Trp Gly Gln Gly Thr Ser Val Thr Val Ser Ser
115 120

<210> 11
<211> 107
<212> PRT
<213> Artificial

<220>
<223> nonS_RA15-7VL_AA

<400> 11

Asp Ile Gln Met Thr Gln Ser Pro Ala Ser Leu Ser Ala Ser Leu Gly
1 5 10 15

Glu Thr Val Thr Ile Glu Cys Arg Thr Ser Glu Asp Ile Phe Arg Asn
20 25 30

Leu Ala Trp Tyr Gln Gln Arg Pro Gly Asn Ser Pro Gln Leu Leu Ile
35 40 45

Tyr Asp Thr Asn Arg Leu Ala Asp Gly Val Pro Ser Arg Phe Ser Gly
50 55 60

Ser Gly Ser Gly Thr Gln Tyr Ser Leu Lys Ile Asn Ser Leu Gln Ser
65 70 75 80

Glu Asp Val Ala Gly Tyr Phe Cys Gln Gln Tyr Asp Asn Tyr Pro Leu
85 90 95

Thr Phe Gly Ser Gly Thr Lys Leu Glu Ile Lys
100 105

<210> 12
<211> 774
<212> DNA
<213> Homo sapiens

<400> 12
atggctcagc ggatgacaac acagctgctg ctccttctag tgtgggtggc tgtagtaggg 60

gaggctcaga caaggattgc atgggccagg actgagcttc tcaatgtctg catgaacgcc 120

aagcaccaca aggaaaagcc aggccccgag gacaagttgc atgagcagtg tcgaccctgg 180

aggaagaatg cctgctgttc taccaacacc agccaggaag cccataagga tgtttcctac 240

ctatatagat tcaactggaa ccactgtgga gagatggcac ctgcctgcaa acggcatttc 300

atccaggaca cctgcctcta cgagtgctcc cccaacttgg ggccctggat ccagcaggtg 360

gatcagagct ggcgcaaaga gcgggtactg aacgtgcccc tgtgcaaaga ggactgtgag 420

caatggtggg aagattgtcg cacctcctac acctgcaaga gcaactggca caagggctgg 480

aactggactt cagggtttaa caagtgcgca gtgggagctg cctgccaacc tttccatttc 540

tacttcccca cacccactgt tctgtgcaat gaaatctgga ctcactccta caaggtcagc 600

aactacagcc gagggagtgg ccgctgcatc cagatgtggt tcgacccagc ccagggcaac 660

cccaatgagg aggtggcgag gttctatgct gcagccatga gtggggctgg gccctgggca 720

gcctggcctt tcctgcttag cctggcccta atgctgctgt ggctgctcag ctga 774

<210> 13
<211> 13
<212> PRT
<213> Artificial

<220>

<223> HuRA15-7CTAccVHCDR3_AA

<400> 13

```
Thr Leu Tyr Gly Tyr Ala Tyr Tyr Tyr Val Met Asp Ala
1               5                   10
```

## Description

Technical Field

The present invention relates to a method for treating a cancer by using a human FOLR1-targeting drug which binds to FOLR1 and which has anti-tumor activity, wherein the method is characterized by increasing an expression of folate receptor $\alpha$ (hereinafter referred to as FOLR1) with an antagonist for folic acid metabolism. Moreover, the invention relates to a method for enhancing a therapeutic effect of a human FOLR1-targeting drug characterized by increasing an expression of FOLR1 by administering an antagonist for folic acid metabolism simultaneously or sequentially. Furthermore, the invention relates to a medicament for a cancer including a human FOLR1-targeting drug characterized by increasing an expression of FOLR1 with an antagonist for folic acid metabolism and to a medicament for a cancer including a human FOLR1-targeting drug for increasing an expression of FOLR1 by administering an antagonist for folic acid metabolism simultaneously or sequentially.

Background Art

FOLR1 is a GPI-anchored membrane protein having a high affinity for folic acid and has important functions in cell growth and survival (Non-Patent Document 1). FOLR1 expression is normally limited at the luminal surface of such as kidney, lung, intestine or the like (Non-Patent Document 2).

The expression of FOLR1 in cancer tissues, however, is not limited at the luminal surface, and FOLR1 is highly expressed in many types of cancer such as ovarian cancer (Non-Patent Document 2, Non-Patent Document 3 and Non-Patent Document 4), renal cancer (Non-Patent Document 2), lung cancer (Non-Patent Document 2 and Non-Patent Document 3), breast cancer (Non-Patent Document 2) and mesothelioma (Non-Patent Document 4). In particular, it is reported that the expression level of FOLR1 is correlated to the grade of malignancy, the progression and the prognosis in ovarian cancer (Non-Patent Document 5 and Non-Patent Document 6). Furthermore, it is also known that soluble FOLR1 is significantly increased in the serum of ovarian cancer patients as compared to that of healthy individuals (Non-Patent Document 7).

Human FOLR1-targeting drugs include for example antibodies obtained by humanizing LK26 (Patent Document 1 and Patent Document 2), an anti-human FOLR1 antibody MORAb-003 (farletuzumab), a folic acid-toxin conjugate EC-145 and the like. These drugs are under development for non-small cell lung cancer (NSCLC), ovarian cancer and the like. Pemetrexed is used as a drug for treating mesothelioma or NSCLC. Pemetrexed is under development with the indication of ovarian cancer.

Of human FOLR1-targeting drugs, the anti-human FOLR1 antibody MORAb-003 is reported to have responses in some patients with platinum-sensitive recurrent ovarian cancer (Non-Patent Document 8). In contrast, it is reported that the clinical trials thereof have been terminated because MORAb-003 did not show benefit for the patients with platinum-resistant ovarian cancer or NSCLC (Non-Patent Document 9 and Non-Patent Document 10). It is reported that the folic acid-toxin conjugate EC-145 showed the responses by combination with Doxil (R) (liposomal doxorubicin) for the patients with ovarian cancer, and by combination with docetaxel in NSCLC when the patients who have the cancer with high FOLR1 expression level and high folic acid uptake activity (Non-Patent Document 11 and Non-Patent Document 12).

Prior Art Document

Patent Document

Patent Document 1: European Patent Application Publication No. 0197435
Patent Document 2: US Patent No. 5952484

Non-Patent Document

Non-Patent Document 1: Int J Cancer, 2006.119(2): p.243-50.
Non-Patent Document 2: Anal Biochem, 2005. 338(2): p.284-93.
Non-Patent Document 3: J Thorac Oncol, 2012.7(5): p.833-840.
Non-Patent Document 4: J Thorac Cardiovasc Surg, 2001.121(2): p.225-33.

Non-Patent Document 5: Int J Cancer, 1997.74(2): p.193-8.
Non-Patent Document 6: Int J Cancer, 1998.79(2): p.121-6.
Non-Patent Document 7: PLoS One, 2009.4(7): p.e6292.
Non-Patent Document 8: News Release, No.13-05, Jan 11, 2013, Eisai Co., Ltd. (http://www.eisai.com/news/news201305.html)
Non-Patent Document 9: News Release, December 2, 2011, Morphotek Inc. (http://www.morphotek.com/news-events/News-Archive/2011-News/Morphotek-Terminates-Farletuzumab-Study-(FAR-122)-.aspx)
Non-Patent Document 10: Maltzman, J. D. et al. 15th World Conf Lung Cancer (Oct 27-30, Sydney) 2013, Abst P1.11-019
Non-Patent Document 11: J Clin Oncol 31:4400-4406 (2013)
Non-Patent Document 12: PRESS RELEASES, Mar 21,2014, Endocyte, Inc. (http://investor.endocyte.com/releasedetail.cfm?ReleaseID=834444)

Summary of Invention

Problems to Be Solved by the Invention

Therapeutic methods for solid cancers and improvement of the prognosis thereof are making rapid progress. However, the prognosis of ovarian cancer has not been improved remarkably since the therapeutic method using a platinum-based drug has been introduced in the 1980s [Nat Rev Cancer, 2011.11(10): p.719-25.], and ovarian cancer is still a leading cause of death among gynecologic cancer. The reason of treatment difficulty is caused by platinum resistance of ovarian cancer after recurrence. Therefore, prolongation of the recurrent free period after response to the chemotherapies such as a platinum-based therapy, inhibition of acquired platinum resistance of ovarian cancer, and establishment of a novel method for treating ovarian cancer which has become resistant to platinum-based chemotherapies, are challenges in the treatment of ovarian cancer.
Human FOLR1-targeting drugs are under development as a therapeutic drug for ovarian cancer. Sufficient expression of FOLR1 in the cancer cells is believed to be important for the treatment efficacy of the human FOLR1-targeting drugs as an anti-tumor drug. Therefore, a method for increasing the expression of FOLR1 in cancer cells for further improving an anti-tumor effect of a human FOLR1-targeting drug is required.
The invention provides a method for improving an anti-tumor effect of human FOLR1-targeting drugs by increasing an expression of FOLR1 in cancer cells with an antagonist for folic acid metabolism. For example, this method is capable of causing a higher therapeutic effect of a human FOLR1-targeting drug on a cancer, in which an expression level of FOLR1 is low and not sufficient for an anti-tumor activity of the human FOLR1-targeting drug, by administering an antagonist for folic acid metabolism simultaneously or sequentially and thereby increasing an expression of FOLR1.

Means for Solving the Problems

The present invention relates to the following [1] to [18].

[1] A method for treating a cancer by using a human FOLR1-targeting drug, wherein an expression of folate receptor $\alpha$ (hereinafter abbreviated to FOLR1) is increased by using an antagonist for folic acid metabolism.
[2] The method according to [1], wherein the antagonist for folic acid metabolism is administered simultaneously or sequentially.
[3] The method according to [1] or [2], wherein the cancer is a cancer which expresses FOLR1.
[4] The method according to any one of [1] to [3], wherein the human FOLR1-targeting drug is any one selected from a FOLR1 inhibitor, an anti-human FOLR1 antibody and an anti-human FOLR1 antibody fragment.
[5] The method according to [4], wherein the FOLR1 inhibitor is a folic acid derivative.
[6] The method according to [4], wherein the anti-human FOLR1 antibody and the antibody fragment are an antibody and a fragment of the antibody which bind to an epitope contained in the amino acid sequence of from position 55 to position 62 in the amino acid sequence of SEQ ID NO: 1.
[7] A method for enhancing a therapeutic effect of a human FOLR1-targeting drug by increasing an expression of FOLR1 in a cancer cell by using an antagonist for folic acid metabolism.
[8] The method according to [7], wherein the antagonist for folic acid metabolism is administered simultaneously or sequentially.
[9] The method according to [7] or [8], wherein the cancer is a cancer which expresses FOLR1.
[10] The method according to any one of [7] to [9], wherein the human FOLR1-targeting drug is any one selected from a FOLR1 inhibitor, an anti-human FOLR1 antibody and an anti-human FOLR1 antibody fragment.
[11] The method according to [10], wherein the FOLR1 inhibitor is a folic acid derivative.

[12] The method according to [10], wherein the anti-human FOLR1 antibody and the antibody fragment are an antibody and a fragment of the antibody which bind to an epitope contained in the amino acid sequence of from position 55 to position 62 in the amino acid sequence of SEQ ID NO: 1.
[13] A medicament for a cancer containing a human FOLR1-targeting drug for increasing an expression of FOLR1 in a cancer cell by administering an antagonist for folic acid metabolism.
[14] The medicament according to [13], wherein the antagonist for folic acid metabolism is administered simultaneously or sequentially.
[15] The medicament according to [13] or [14], wherein the cancer is a cancer which expresses FOLR1.
[16] The medicament according to any one of [13] to [15], wherein the human FOLR1-targeting drug is any one selected from a FOLR1 inhibitor, an anti-human FOLR1 antibody and an anti-human FOLR1 antibody fragment.
[17] The medicament according to [16], wherein the FOLR1 inhibitor is a folic acid derivative.
[18] The medicament according to [16], wherein the anti-human FOLR1 antibody and the antibody fragment are an antibody and a fragment of the antibody which bind to an epitope contained in the amino acid sequence of from position 55 to position 62 in the amino acid sequence of SEQ ID NO: 1.

Brief Description of Drawings

[Fig. 1] Fig. 1(A) to Fig. 1(D) show the expression levels of FOLR1 in NSCLC cell lines [NCI-H1437 [Fig. 1(A)], NCI-H2228 [Fig. 1(B)],NCI-H522 [Fig. 1(C)] and NCI-H838 [Fig. 1(D)]] after incubation with pemetrexed. The vertical axis shows the relative expression level of FOLR1, where the expression level under the conditions of cultivation in a low folic acid medium (0 nmol/L pemetrexed) is considered to be 100. The horizontal axis shows the pemetrexed concentration (10 or 1000 nmol/L) in the low folic acid medium.
[Fig. 2] Fig. 2(A) to Fig. 2(D) show the expression levels of FOLR1 in ovarian cancer cell lines [IGR-OV1 [Fig. 2(A)], SKOV-3 [Fig. 2(B)], OVCAR-3 [Fig. 2(C)] and MCAS [Fig. 2(D)]] after incubation with pemetrexed. The vertical axis shows the relative expression level of FOLR1, where the expression level under the conditions of cultivation in a culture medium (NC) is considered to be 100. The horizontal axis shows the culture conditions and shows the culture medium (NC) or the pemetrexed concentration (0, 10 or 1000 nmol/L) in the low folic acid medium.
[Fig. 3] Fig. 3(A) and Fig. 3(B) show the ADCC activities against MCAS using PBMCs derived from a healthy donor 1 [Fig. 3(A)] and a donor 2 [Fig. 3(B)]. The lines with black circles, white circles, black triangles and white triangles show the ADCC activity of HuRA15-7CTAcc antibody against MCAS cultured in the presence of 20 nmol/L pemetrexed, the ADCC activity of HuRA15-7CTAcc antibody against MCAS cultured in the absence of pemetrexed, the ADCC activity of MORAb-003 antibody against MCAS cultured in the presence of 20 nmol/L pemetrexed, and the ADCC activity of MORAb-003 antibody against MCAS cultured in the absence of pemetrexed, respectively. The vertical axis shows the ADCC activity (%), and the horizontal axis shows the antibody concentration (ng/mL). Asterisk (*) indicates that the value of the black circle is significant (P<0.05) as compared to the value of the white circle at the antibody concentration, and two asterisks (**) indicates that the value of the black triangle is significant (P<0.05) as compared to the value of the white triangle at the antibody concentration. To test the significance of difference, 1-way ANOVA-Dunnett test was used.
[Fig. 4] Fig. 4(A) to Fig. 4(D) show the results of the combination use of pemetrexed and an anti-human FOLR1 antibody in an SCID mouse model subcutaneously transplanted ovarian cancer cell line MCAS. The vertical axes of Fig. 4(A) to Fig. 4(D) show the tumor volume ($mm^3$), V/V0, T/C, and the body weight (g), respectively. The horizontal axes of Fig. 4(A) to Fig. 4(D) show the days after started the treatments. The lines with white circles, black circles, white triangles, black diamonds and white squares show the results of the vehicle administration group, the HuRA15-7CTAcc antibody single administration group, the pemetrexed single administration group, the simultaneous combined use group, and the sequential combined use group, respectively.
[Fig. 5] Fig. 5(A) to Fig. 5(C) show the expression levels of FOLR1 in endometrial cancer cell lines [MES-SA [Fig. 5(A)], HEC-1-A [Fig. 5(B)] and HEC-1-B [Fig. 5(C)]] after incubation with pemetrexed. The vertical axis shows the relative expression level of FOLR1, where the expression level under the conditions of cultivation in a culture medium (NC) is considered to be 100. The horizontal axis shows the culture conditions and shows the culture medium (NC) or the pemetrexed concentration (0, 10 or 1000 nmol/L) in the low folic acid medium.
[Fig. 6] Fig. 6(A) and Fig. 6(B) show the ADCC activities of HuRA15-7CTAcc antibody [Fig. 6(A)] and MORAb-003 [Fig. 6(B)] antibody against a NSCLC cell line NCI-H2228 using healthy donor-derived PBMCs. The broken lines with white triangles, the solid lines with white circles and the solid lines with black circles show the ADCC activities against NCI-H2228 cultured in the presence of 0, 20 and 1000 nmol/L pemetrexed, respectively. The vertical axis shows the ADCC activity (%), and the horizontal axis shows the antibody concentration (ng/mL). Asterisk (*) indicates that the value of the solid line with black circles is significant (P<0.05) as compared to the value of the broken line with white triangles at the antibody concentration. To test the significance of difference, 1-way ANOVA-Dunnett test was used.

Modes for Carrying Out the Invention

The therapeutic method of the invention includes a method for treating a cancer by using a human FOLR1-targeting drug in which an expression of FOLR1 is increased by using an antagonist for folic acid metabolism, a method for treating a cancer characterized by increasing an expression of FOLR1 by administering an antagonist for folic acid metabolism and a human FOLR1-targeting drug simultaneously or sequentially and the like.

The method of the invention for enhancing a therapeutic effect includes a method for enhancing a therapeutic effect of a human FOLR1-targeting drug by increasing an expression of FOLR1 in a cancer cell by using an antagonist for folic acid metabolism, a method for enhancing a therapeutic effect of a human FOLR1-targeting drug characterized by increasing an expression of FOLR1 in a cancer cell by administering an antagonist for folic acid metabolism and a human FOLR1-targeting drug simultaneously or sequentially and the like.

The medicament of the invention includes a medicament for a cancer containing a human FOLR1-targeting drug for increasing an expression of FOLR1 by using an antagonist for folic acid metabolism, a medicament for a cancer containing a human FOLR1-targeting drug for increasing an expression of FOLR1 by administering an antagonist for folic acid metabolism and a human FOLR1-targeting drug simultaneously or sequentially and the like.

In the invention, a cancer cell which has been exposed to an antagonist for folic acid metabolism requires more folic acid because metabolic function of a folic acid is decreased. Similarly, a cancer cell in an environment in which concentration of a folic acid is decreased or in which folic acid is depleted requires more folic acid to keep the intracellular folic acid concentration. Accordingly, in a cancer cell which has been exposed to an antagonist for folic acid metabolism or to an environment in which concentration of a folic acid is low or in which a folic acid is depleted, an increase in an expression of FOLR1 following an increase in a transcription of mRNA encoding FOLR1 and an increase an amount of the translated protein or an increase in an expression level of FOLR1 on the cell membrane due to a change in the intracellular dynamics is induced by a compensatory reaction to increase an uptake of folic acid. As a result, a human FOLR1-targeting drug can bind to FOLR1 which is expressed more on the membrane of the cancer cell and can exert a higher therapeutic effect as compared to the effect before the exposure to the antagonist for folic acid metabolism.

Folate receptors (FRs) are glycophosphatidyl inositol (GPI)-anchored membrane-bound glycoproteins. Folate receptors form a family including FOLR1, FOLR2, FOLR3 and FOLR4 (also called FR$\alpha$, FR$\beta$, FR$\gamma$ and FR$\delta$) and are involved in the uptake of oxidized folic acid into the cell. Moreover, folate receptors are known to bind to folic acid at higher affinity than reduced folate carriers (RFCs) which are involved in the uptake of reduced folic acid.

The human FOLR1 in the invention includes a polypeptide which contains the amino acid sequence of SEQ ID NO: 1 or GenBank accession No. NP_057937.1 and which has a function of human FOLR1, a polypeptide which contains the amino acid sequence of SEQ ID NO: 1 or GenBank accession No. NP_057937.1, wherein one or more amino acids are deleted, replaced or added, and which has the function of human FOLR1, a polypeptide which has an amino acid sequence having homology of 60% or more, preferably 80% or more, further preferably 90% or more with the amino acid sequence of SEQ ID NO: 1 or GenBank accession No. NP_057937.1, a polypeptide which contains an amino acid sequence having homology of most preferably 95% or more and which has the function of human FOLR1, a polypeptide which contains an amino acid sequence comprising a partial sequence of the amino acid sequence of SEQ ID NO: 1 or GenBank accession No. NP_057937.1 and which has the function of human FOLR1 and the like.

The gene encoding human FOLR1 includes a nucleotide sequence of SEQ ID NO: 12 or GenBank accession No. NM_016725. A gene which has the nucleotide sequence of SEQ ID NO: 12 or GenBank accession No. NM_016725, wherein one or more bases are deleted, replaced or added, and which contains DNA encoding a polypeptide having the function of human FOLR1, a gene which has a nucleotide sequence having homology of at least 60% or more with the nucleotide sequence of SEQ ID NO: 12 or GenBank accession No. NM_016725, preferably a nucleotide sequence having homology of 80% or more, further preferably a nucleotide sequence having homology of 95% or more, and which contains DNA encoding a polypeptide having the function of human FOLR1, a gene which contains DNA that hybridizes with DNA having the nucleotide sequence of SEQ ID NO: 12 or GenBank accession No. NM_016725 under stringent conditions and which contains DNA encoding a polypeptide having the function of human FOLR1 and the like are also included in the gene encoding human FOLR1 of the invention.

In the invention, the function of human FOLR1 includes a function of causing endocytosis by binding with a ligand (for example, folic acid) and incorporating folic acid into the cell.

In the invention, the increasing of the expression of FOLR1 means for example: that an expression level of FOLR1 is increased by an exposure to an antagonist for folic acid metabolism in a cancer cell in which the expression level of FOLR1 is low or in which substantially no FOLR1 is expressed; that an expression level of FOLR1 is further increased by an exposure to an antagonist for folic acid metabolism in a cancer cell which originally expresses FOLR1; and that an expression level of FOLR1 is increased by an exposure to an antagonist for folic acid metabolism in the FOLR1-expressing cell and a transport function of a folic acid is promoted.

Such an increase in the expression of FOLR1 is caused in the following cases: an expression of a gene encoding FOLR1 protein is increased; an expression of FOLR1 protein on the cell membrane is increased due to an increase in an expression of FOLR1 protein; or a function of FOLR1 is promoted due to an increase in the expression of highly functional FOLR1 protein following an addition, a deletion, a replacement or the like.

In the invention, the expression of FOLR1 and the increase in the expression can be confirmed for example by reacting labelled folic acid or anti-FOLR1 antibody with a cell isolated and separated from a lesion tissue or a cancer tissue and then measuring with a flow cytometer (FCM) or the like.

Moreover, the expression of FOLR1 and the increase in the expression in a tissue can be confirmed by immunohistochemistry (IHC) or the like. Also, the expression of FOLR1 in the tissue, the increase in the expression and a promotion of the function of FOLR1 can be confirmed by administering folic acid conjugated to a radioisotope or the like to a body of a patient and observing an uptake of folic acid into a tissue.

The antagonist for folic acid metabolism used in the invention is incorporated into a cell through a reduced folate carrier (RFC), a folate receptor family (FOLR1, FOLR2, FOLR3 and FOLR4), a proton-coupled folate transporter (PCFT) or the like.

In a cell, the antagonist for folic acid metabolism inhibits a folic acid metabolic pathway of the cell by inhibiting dihydrofolate reductase (DHFR), thymidylate synthase (TS), glycineamide ribonucleotide transformylase (GARTF) and the like and exerts cell growth-inhibiting activity, cellular cytotoxicity, apoptotic activity and/or anti-tumor activity (Cancer Metastasis Rev (2007)26:153-181). Accordingly, the antagonist for folic acid metabolism used for the therapeutic method and the medicament of the invention may be any drug and is not limited as long as an antagonist acts on the mechanism of folic acid metabolism.

Specific examples of the antagonist for folic acid metabolism used in the invention are pemetrexed [Drug Metab Dispos. 1997 Jun;25(6):693-700.], which is the compound of CAS No. 357166-29-1, methotrexate, raltirexed, edatorexate, trimetrexate, pralatrexate, aminopterin, lometrexol, nolatrexed, PT523 and the like.

The human FOLR1-targeting drug used in the invention may be any drug which binds to human FOLR1 and which has cell growth-inhibiting activity, cellular cytotoxicity, apoptotic activity, anti-tumor activity and/or the like, and the human FOLR1-targeting drug may be a low molecular weight or high molecular weight drug.

In the invention, binding of the human FOLR1-targeting drug to human FOLR1 means that the human FOLR1-targeting drug recognizes and binds to an amino acid sequence of human FOLR1 or a conformation thereof. That is, the human FOLR1-targeting drug acts by recognizing and binding to human FOLR1 having a native conformation which is expressed on a cell membrane of a cancer cell.

An example of the low molecular weight human FOLR1-targeting drug is a FOLR1 inhibitor. The FOLR1 inhibitor may be any FOLR1 inhibitor as long as the FOLR1 inhibitor has FOLR1-binding activity and has cell growth-inhibiting activity, cytotoxicity, apoptotic activity and/or anti-tumor activity, such as folic acid, a folic acid derivative having FOLR1-binding activity, a folic acid-toxin conjugate and a folic acid-radioisotope conjugate.

Examples thereof include vintafolide (EC-145), $^{99m}Tc(CO)_3$-folate, $^{99m}$Tc-EC20 (etarfolatide), $^{111}$In-DTPA-folate, $^{111}$In/$^{177}$Lu-DOTA-click-folate, $^{67}$Ga-DOTA-Bz-folate ($^{67}$Ga-EC0800), $^{67}$Ga-NODAGA-folate and the like.

EC-145 is a compound obtained by binding a tubulin polymerization inhibitor (desacetylvinblastine monohydrazide) to folic acid [Curr Opin Investig Drugs. 2010 Dec; 11(12):1424-33.], and EC-145 binds to human FOLR1 and has cell growth-inhibiting activity and anti-tumor activity.

The cell growth-inhibiting activity refers to an activity of inhibiting a cell growth of a FOLR1 positive cell and is caused by various mechanisms, such as a direct inhibition of the cell growth due to specific binding of folic acid, a folic acid derivative and a folic acid conjugate to FOLR1, an induction of apoptotic activity and cellular cytotoxicity of conjugated toxin, radioisotope or the like.

The anti-tumor activity refers to an activity of inhibiting a growth of a FOLR1 positive tumor cell and is caused by the mechanisms described above.

The apoptotic activity means that programmed cell death is caused as a result of an increase in caspase activity due to binding of folic acid, a folic acid derivative and a folic acid conjugate to a FOLR1 positive cell.

Examples of the high molecular weight human FOLR1-targeting drug include an anti-human FOLR1 antibody and an anti-human FOLR1 antibody fragment.

The anti-human FOLR1 antibody used for the therapeutic method and the medicament of the invention may be any of a polyclonal antibody, an oligoclonal antibody and a monoclonal antibody as long as the anti-human FOLR1 antibody is an antibody which recognizes and binds to the amino acid sequence of human FOLR1 or the conformation formed by the amino acid sequence, but a monoclonal antibody is preferable.

A monoclonal antibody is an antibody secreted by antibody-producing cells of a single clone and is characterized by recognizing only one epitope (also called an antigenic determinant) and characterized in that an amino acid sequence (primary structure) composing the monoclonal antibody is uniform. An oligoclonal antibody is an antibody composition containing more than one monoclonal antibody and is an antibody containing several to around 10 monoclonal antibodies.

A polyclonal antibody is an antibody composition containing more than one monoclonal antibody and is a mixture of antibodies binding to more than one epitope on an antigen.

The epitope in the invention includes a single amino acid sequence, a conformation formed by an amino acid sequence, a sugar chain-bound amino acid sequence, a conformation formed by a sugar chain-bound amino acid sequence and the like which a monoclonal antibody recognizes and binds to. Accordingly, the epitope may be any epitope as long as the epitope is present on a human FOLR1 molecule expressed on the cell membrane of a cancer cell, but the amino acid sequence of from position 55 to position 62 in the amino acid sequence of human FOLR1 of SEQ ID NO: 1 (NCBI Reference Sequence: NP_057937.1) is preferable.

In the invention, the epitope to which the anti-human FOLR1 antibody binds is more preferably an epitope containing at least one amino acid residue selected from the amino acid sequence of from position 55 to position 62 in the amino acid sequence of SEQ ID NO: 1, more preferably an epitope containing at least two or more consecutive amino acid residues selected from the amino acid sequence of from position 55 to position 62 in the amino acid sequence of SEQ ID NO: 1 of the amino acid sequence of human FOLR1.

The conformation of human FOLR1 in the invention may be any structure as long as a conformation has an equivalent structure to a structure which human FOLR1 containing the amino acid sequence of from position 1 to position 257 in the amino acid sequence of SEQ ID NO: 1 can have in the native state. The conformation which human FOLR1 can have in the native state means a native conformation which human FOLR1 expressed on a cell membrane has.

The monoclonal antibody used in the invention can include an antibody produced by a hybridoma or a recombinant antibody produced by a transformant transformed by an expression vector containing the antibody gene.

The hybridoma can be prepared for example by preparing human FOLR1 protein, a human FOLR1 protein fragment, an oligopeptide of human FOLR1, a cell expressing human FOLR1 or the like as the antigen, inducing an antibody-producing cell having antigen specificity in an animal immunized with the antigen and further fusing the antibody-producing cell with a myeloma cell. An anti-FOLR1 monoclonal antibody can be obtained by culturing the hybridoma or causing ascites carcinoma in an animal by administering the hybridoma cells to the animal and then separating and purifying the culture solution or the ascites.

The recombinant antibody used in the invention includes antibodies produced by genetic recombination, such as a human chimeric antibody, a human CDR-grafted antibody (also called a humanized antibody), a human antibody or fragments of the antibodies. A recombinant antibody which has characteristics of monoclonal antibodies, low antigenicity and an extended half-life in blood is preferable as a therapeutic drug. An example of the recombinant antibody is an antibody obtained by modifying a monoclonal antibody produced by the hybridoma using genetic recombination.

A human chimeric antibody is an antibody having VH and VL of an antibody of a non-human animal and a heavy chain (hereinafter abbreviated to H chain) constant region (hereinafter referred to as CH) and a light chain (hereinafter abbreviated to L chain) constant region (hereinafter referred to as CL) of a human antibody. The human chimeric antibody of the invention can be produced by obtaining cDNAs encoding VH and VL from a hybridoma producing a monoclonal antibody which specifically recognizes and binds to the amino acid sequence of human FOLR1 or the conformation thereof, inserting the cDNAs respectively into an expression vector for animal cells having genes encoding CH and CL of a human antibody to construct a human chimeric antibody expression vector, introducing the expression vector to an animal cell and expressing the antibody.

The CH of the human chimeric antibody may be any CH as long as a CH belongs to human immunoglobulin (hereinafter referred to as hIg). CH of the class hIgG is preferably used, and any CH of the subclasses belonging to the class hIgG such as hIgG1, hIgG2, hIgG3 or hIgG4 can be used. Moreover, the CL of the human chimeric antibody may be any CL as long as a CL belongs to hIg, and CL of the class κ or the class λ can be used.

A specific example of the human chimeric antibody used in the invention is a chimeric antibody which contains VH of an antibody containing the amino acid sequence of SEQ ID NO: 10 and which contains VL of an antibody containing the amino acid sequence of SEQ ID NO: 11.

The chimeric antibody used in the invention also includes a chimeric antibody which competes with the monoclonal antibody and which specifically recognizes and binds to the amino acid sequence of human FOLR1 or the conformation thereof and a chimeric antibody which binds to the same epitope as the epitope in human FOLR1 to which the monoclonal antibody binds.

The human complementarity determining region (hereinafter abbreviated to CDR)-grafted antibody (also called a humanized antibody) used in the invention is an antibody obtained by grafting an amino acid sequences of CDRs of VH and VL of an antibody of a non-human animal at appropriate positions of VH and VL of a human antibody.

The human CDR-grafted antibody used in the invention can be produced by constructing cDNAs encoding V regions in which an amino acid sequences of CDRs of VH and VL of a monoclonal antibody of a non-human animal which specifically recognizes and binds to the amino acid sequence of human FOLR1 or the conformation thereof are grafted to the framework regions (hereinafter referred to as FRs) of VH and VL of any human antibody, inserting the cDNAs respectively into an expression vector for animal cells having genes encoding CH and CL of a human antibody to construct a human CDR-grafted antibody expression vector, introducing the expression vector to an animal cell and expressing the antibody.

The CH of the human CDR-grafted antibody may be any CH as long as a CH belongs to hIg. CH of the class hIgG is preferably used, and any CH of the subclasses belonging to the class hIgG such as hIgG1, hIgG2, hIgG3 or hIgG4 can be used. Moreover, the CL of the human CDR-grafted antibody may be any CL as long as a CL belongs to hIg, and CL of the class $\kappa$ or the class $\lambda$ can be used.

A human antibody originally means an antibody which is present naturally in the human body, but antibodies obtained from a human antibody phage library and a human antibody-producing transgenic animal which have been produced by the recent progress in the technology of genetic engineering, cell engineering and development engineering and the like are also included.

Specific examples of the human CDR-grafted antibody or the human antibody used in the invention include: a humanized antibody which contains VH of an antibody having CDRs 1 to 3 respectively containing the amino acid sequences of SEQ ID NOs: 2 to 4 and which contains VL of an antibody having CDRs 1 to 3 respectively containing the amino acid sequences of SEQ ID NOs: 5 to 7; a humanized antibody which contains the H chain of an antibody having CDRs 1 to 3 respectively containing the amino acid sequences of SEQ ID NOs: 2 to 4 and the L chain of an antibody having CDRs 1 to 3 respectively containing the amino acid sequences of SEQ ID NOs: 5 to 7 and in which the cysteine in the amino acid sequence of SEQ ID NO: 4 (CDR3 of the antibody H chain) is replaced with threonine, methionine, isoleucine, valine, phenylalanine or glutamine; and a humanized antibody which contains VH of an antibody having CDRs 1 to 3 respectively containing the amino acid sequences of SEQ ID NOs: 2,3 and 13 and which contains VL of an antibody having CDRs 1 to 3 respectively containing the amino acid sequences of SEQ ID NOs: 5, 6 and 7.

Moreover, a more specific example of the humanized antibody used in the invention is a humanized antibody in which VH of the antibody contains the amino acid sequence of SEQ ID NO: 8 and/or in which VL of the antibody contains the amino acid sequence of SEQ ID NO: 9 (HuRA15-7CTAcc antibody).

The anti-human FOLR1 antibody used in the invention also includes an antibody which competes with any antibody of the monoclonal antibody and the recombinant antibody described above and the like and which specifically recognizes and binds to the amino acid sequence of human FOLR1 or the conformation thereof, a fragment of the antibody, a monoclonal antibody which binds to the same epitope as the epitope in human FOLR1 to which the monoclonal antibody binds, a fragment of the antibody and the like.

The "antibody which competes with the monoclonal antibody" used in the invention refers to an antibody which binds to the same or partially same epitope (also called an antigenic determinant) as that of the anti-human FOLR1 antibody used in the invention in human FOLR1. The "antibody which binds to the same epitope as the epitope to which the anti-human FOLR1 antibody binds" used in the invention refers to an antibody which recognizes and binds to the same sequence as the amino acid sequence of human FOLR1 which the anti-human FOLR1 antibody used in the invention recognizes.

The antibody fragment used in the invention includes Fab, $F(ab')_2$, Fab', a single-chain antibody (scFv), a dimeric V region (diabody), a disulfide-stabilized V region (dsFv), a peptide containing CDRs 1-3 of the H chain and CDRs 1-3 of the L chain of a monoclonal antibody, a peptide containing a CDR and the like.

Moreover, the antibody fragment used in the invention also includes any antibody fragments having FOLR1-binding activity, such as a bispecific antibody, a trispecific antibody, a tetraspecific antibody, a Fc-fusion peptide and a monovalent antibody having Fc-fusion L chain and H chain which contain an antibody fragment.

That is, the anti-human FOLR1 antibody used in the invention includes the anti-FOLR1 antibodies of (i) to (viii) below and fragments of the antibodies.

(i) An antibody which binds to an epitope contained in the amino acid sequence of from position 55 to position 62 in the amino acid sequence of SEQ ID NO: 1.

(ii) An antibody which binds to at least two or more consecutive amino acid residues contained in the amino acid sequence of from position 55 to position 62 in the amino acid sequence of SEQ ID NO: 1.

(iii) An antibody which binds to an epitope containing the amino acid sequence of from position 55 to position 62 in the amino acid sequence of SEQ ID NO: 1.

(iv) An antibody which contains the H chain of an antibody having CDRs 1 to 3 respectively containing the amino acid sequences of SEQ ID NOs: 2, 3 and 4 and which contains the L chain of an antibody having CDRs 1 to 3 respectively containing the amino acid sequences of SEQ ID NOs: 5, 6 and 7.

(v) An antibody which contains the H chain of an antibody having CDRs 1 to 3 respectively containing the amino acid sequences of SEQ ID NOs: 2, 3 and 4 and the L chain of an antibody having CDRs 1 to 3 respectively containing the amino acid sequences of SEQ ID NOs: 5, 6 and 7 and in which the cysteine in the amino acid sequence of SEQ ID NO: 4 (CDR3 of the antibody H chain) is replaced with threonine, methionine, isoleucine, valine, phenylalanine or glutamine.

(vi) An antibody which contains the H chain of an antibody having CDRs 1 to 3 respectively containing the amino acid sequences of SEQ ID NOs: 2, 3 and 13 and which contains the L chain of an antibody having CDRs 1 to 3 respectively containing the amino acid sequences of SEQ ID NOs: 5, 6 and 7.

(vii) An antibody which competes with any of the antibodies of (i) to (vi) or a fragment of the antibodies and which binds to human FOLR1 .

(viii) An antibody which binds to the same epitope as the epitope which any of the antibodies of (i) to (vii) or a fragment of the antibodies recognizes.

In addition, the anti-human FOLR1 antibody used in the invention includes farletuzumab (MORAb-003) and the like. MORAb-003 is an anti-human FOLR1 humanized IgG1 antibody and has anti-tumor activity against a FOLR1 positive cancer mainly due to antibody-dependent cellular cytotoxicity (hereinafter abbreviated to ADCC activity) and complement-dependent cytotoxicity (hereinafter abbreviated to CDC activity) (Cancer Immun. 2007 Mar 9;7:6.).

The anti-human FOLR1 antibody used in the invention also includes an antibody in which one or more amino acids of the amino acid sequence composing any of the antibodies described above or a fragment of the antibodies are deleted, added, replaced or inserted and which has a similar activity to that of any of the antibodies described above or a fragment of the antibodies, or a fragment of the antibody.

Furthermore, as the anti-human FOLR1 antibody used in the invention, a derivative of an anti-human FOLR1 antibody obtained by binding a radioactive isotope such as $^{64}Cu$, $^{67}Ga$, $^{90}Y$, $^{99}Tc$, $^{111}In$, $^{125}I$, $^{131}I$, $^{177}Lu$ and $^{211}At$, an anticancer agent, a low molecular weight drug such as toxin, a high molecular weight drug, a protein, an antibody medicament or the like by chemical or genetic engineering technique to a monoclonal antibody which specifically recognizes and binds to the amino acid sequence of human FOLR1 or the conformation thereof, or a fragment of the antibody can also be used.

In the invention, recognizing and binding of the human FOLR1-targeting drug to the amino acid sequence of human FOLR1 or the conformation thereof can be confirmed by a method which examines the affinity of a specific antigen and a low molecular weight compound or a high molecular weight compound such as an antibody to a specific antigen, such as enzyme-linked immunosorbent assay (ELISA) using immobilized human FOLR1, western blot, immunohistochemistry (IHC), a known immunological detecting method using a cell expressing human FOLR1 and fluorescent cell staining. Specifically, methods such as fluorescent antibody staining using FMAT8100HTS system (manufactured by Applied Biosystems) or the like [Cancer Immunol. Immunother., 36, 373(1993)], fluorescent cell staining using flow cytometry, surface plasmon resonance (SPR) using Biacore system (manufactured by GE Healthcare) or the like and isothermal titration calorimetry using ITC (manufactured by DKSH) or the like are used.

For example, binding of the anti-human FOLR1 antibody to the amino acid sequence of human FOLR1 or the conformation thereof can be confirmed by surface plasmon resonance using Biacore system (manufactured by GE Healthcare) or the like. Specifically, after immobilizing an anti-IgG antibody on a sensor tip CM5 by amine coupling, the antibody or a fragment of the antibody is caused to flow, appropriate amount of the antibody or a fragment of the antibody is caused to bind, and then FOLR1 or a cointegrate thereof having known concentrations is caused to flow. Then, the binding and the dissociation can be measured.

The anti-human FOLR1 antibody used in the invention includes an antibody having cell growth-inhibiting activity, anti-tumor activity, apoptotic activity, an effector activity such as ADCC activity, CDC activity and antibody-dependent phagocytotic activity (hereinafter abbreviated to ADP activity) or the like.

The cell growth-inhibiting activity refers to an activity of inhibiting a cell growth of a FOLR1 positive cell and is caused by various mechanisms, such as a direct inhibition of the cell growth due to specific binding of the anti-human FOLR1 antibody to FOLR1, ADCC activity, CDC activity, ADP activity, apoptotic activity and cellular cytotoxicity due to an antibody conjugate.

The anti-tumor activity refers to an activity of inhibiting a growth of a FOLR1 positive tumor cell and is caused by the mechanisms described above.

The apoptotic activity means that programmed cell death is caused as a result of an increase in caspase activity due to binding of the anti-human FOLR1 antibody, the antibody fragment or the antibody conjugate to a FOLR1 positive cell.

The ADCC activity refers to cell lysis caused by a cytotoxic molecule such as perforin or granzyme which is released from a natural killer cell (hereinafter referred to as an NK cell) when an antibody bound to human FOLR1 on a cell surface binds to FcγRIIIa mainly on the NK cell surface through the Fc part [Clark M, Chemical Immunology, 65, 88(1997); Gorter A et al., Immunol. Today, 20, 576(1999)].

The CDC activity refers to a reaction in which an antibody bound to human FOLR1 on a cell surface binds to C1q, a component of C1 of a complement system, through a Fc part and as a result activates a complement components of C1 to C9 and in which C5 to C9 finally form a pore-forming polymer called a membrane attack complex on the cell membrane and causes cell lysis [Immunol Today. 1999 Dec;20(12):576-82.].

The effector activity of the anti-human FOLR1 antibody used in the invention can be regulated. The regulating method includes a method in which the amount of fucose (also called core fucose) linked α-1,6 to N-acetylglucosamine (GlcNAc) at the reducing terminal of a complex-type N-linked sugar chain bound to asparagine (Asn) at position 297 of the Fc region of the antibody is controlled (WO2005/035586, WO2002/31140 and WO00/61739), a regulating method in which an amino acid residue in the Fc region of the antibody is modified or the like.

An example of the method for increasing or decreasing the effector activity of the antibody is a method in which an amount of core fucose of a complex-type N-linked sugar chain of Fc of the antibody is controlled.

An example of the method for decreasing the amount of fucose linked to a complex-type N-linked sugar chain bound to Fc of the antibody is a method in which an antibody to which fucose is not bound is obtained by expressing the antibody using a CHO cell lacking $\alpha$1,6-fucosetransferase gene. The antibody to which fucose is not bound has high ADCC activity.

An example of the method for increasing the amount of fucose linked to a complex-type N-linked sugar chain bound to Fc of the antibody is a method in which an antibody to which fucose is bound is obtained by expressing the antibody using a host cell to which $\alpha$1,6-fucosetransferase gene has been introduced. The antibody to which fucose is bound has lower ADCC activity than the antibody to which fucose is not bound.

Moreover, the ADCC activity or the CDC activity can be increased or decreased by modifying an amino acid residue of a Fc region of an antibody. For example, the CDC activity of the antibody can be increased using the amino acid sequence of the Fc region described in US patent application publication No. 2007/0148165. Also, through the amino acid modifications described in US patent No. 6, 737,056, US patent No. 7, 297, 775 or US patent No. 7, 317, 091, the ADCC activity or the CDC activity can be increased or decreased. Furthermore, when a combination of the methods described above is applied to an antibody, an antibody in which an effector activity of the antibody is regulated can be obtained.

The method for enhancing a therapeutic effect of a human FOLR1-targeting drug of the invention includes a method for enhancing a therapeutic effect of a human FOLR1-targeting drug as a result of simultaneous or sequential administration of an antagonist for folic acid metabolism and the human FOLR1-targeting drug. As a result of an increase in an expression of FOLR1 on a cell membrane of a cancer cell exposed to the antagonist for folic acid metabolism, the human FOLR1-targeting drug binds to FOLR1 expressed at an increased level on the cancer cell membrane and exerts the efficacy. The therapeutic effect of the human FOLR1-targeting drug obtained when the antagonist for folic acid metabolism is applied is higher than the therapeutic effect of the human FLOR1-targeting drug obtained when the antagonist for folic acid metabolism is not applied.

The combined use method of the invention includes a method in which an antagonist for folic acid metabolism is used with a human FOLR1-targeting drug and which is characterized by increasing the expression of FOLR1 by using the antagonist for folic acid metabolism and a method in which an antagonist for folic acid metabolism is used with a human FOLR1-targeting drug and which is characterized by increasing the expression of FOLR1 by administering the antagonist for folic acid metabolism simultaneously or sequentially.

The invention also includes a method for increasing the ADCC activity and/or the anti-tumor activity of an anti-human FLOR1 antibody. As a result of an increase in the expression of FOLR1 on a cell membrane of a cancer cell exposed to the antagonist for folic acid metabolism, the anti-human FOLR1 antibody binds to FOLR1 expressed at an increased level on the cancer cell membrane and exerts high ADCC activity and/or high anti-tumor activity. The ADCC activity and the anti-tumor activity of the human FOLR1-targeting drug obtained when the antagonist for folic acid metabolism is applied are higher than the ADCC activity and the anti-tumor activity of the human FLOR1-targeting drug obtained when the antagonist for folic acid metabolism is not applied.

The medicament containing a human FOLR1-targeting drug of the invention includes a medicament for a cancer containing a human FOLR1-targeting drug characterized by increasing an expression of FOLR1 by using an antagonist for folic acid metabolism, a medicament for a cancer containing a human FOLR1-targeting drug for increasing an expression of FOLR1 by administering an antagonist for folic acid metabolism and an human FOLR1-targeting drug simultaneously or sequentially and the like.

Accordingly, the medicament of the invention is a medicament for a cancer in which an effect of the human FOLR1-targeting drug is enhanced by increasing the expression of FOLR1 by using the antagonist for folic acid metabolism, and the medicament may be for administering the antagonist for folic acid metabolism and the human FOLR1-targeting drug simultaneously or sequentially as long as the expression of FOLR1 can be increased.

Moreover, with respect to the medicament of the invention, the antagonist for folic acid metabolism and the human FOLR1-targeting drug may be contained in separate preparations as more than one preparation or may be contained in a same preparation as a single preparation.

Furthermore, the medicament of the invention may contain one or more pharmacologically acceptable carriers in addition to the antagonist for folic acid metabolism and/or the human FOLR1-targeting drug or a derivative thereof.

The simultaneous use in the therapeutic method, the method for enhancing a therapeutic effect and the medicament of the invention indicates a state in which the human FOLR1-targeting drug and the antagonist for folic acid metabolism are administered to a patient substantially simultaneously. Specifically, a case in which the human FOLR1-targeting drug and the antagonist for folic acid metabolism are administered substantially simultaneously for example by preparing them in one preparation or by administering two or more preparations one by one and the like are included.

The sequential use in the invention indicates a state in which the human FOLR1-targeting drug and the antagonist for folic acid metabolism are administered to a patient in any order, and the frequency of administration of the human FOLR1-targeting drug and that of the antagonist for folic acid metabolism may be the same or different. Specifically, a case in which one is administered first and then only the other one is administered to a patient more than once at certain intervals and the like are included.

The target disease of the therapeutic method, the method for enhancing a therapeutic effect and the medicament of the invention may be any of benign tumors, malignant tumors, cancers as well as continuous metastasis, hematogenous metastasis, lymphatic metastasis and dissemination of primary carcinoma, as long as a FOLR1 positive cell is involved in the disease.

The disease in which a FOLR1 positive cell is involved may be any disease as long as a cell expressing FOLR1 is involved and abnormality of folic acid metabolism and/or abnormality of a folate receptor are involved in the disease, and an example thereof is a cancer.

Examples of the cancer include blood cancer, breast cancer, uterine cancer, large bowel cancer, esophageal cancer, stomach cancer, ovarian cancer, lung cancer, renal cancer, rectal cancer, thyroid cancer, uterine cervix cancer, small intestinal cancer, prostate cancer, mesothelioma, pancreatic cancer and the like, and ovarian cancer, renal cancer, lung cancer, breast cancer, pancreatic cancer or mesothelioma is preferable. The therapeutic method and the medicament of the invention can be used also to improve the symptoms of related syndromes such as metastasis of the cancers, cachexia, cancer pain and cancer neuroses.

In the invention, whether a FOLR1 positive cell is involved in a disease can be determined by observing the FOLR1 positive cell in a lesion tissue. A FOLR1 positive cell can be observed by causing labelled folic acid or anti-FOLR1 antibody to react with a cell isolated and separated from a lesion tissue or a cancer tissue and then measuring with a flow cytometer (FCM) or the like. Also, the expression of FOLR1 in a tissue can be also observed by immunohistochemistry (IHC). Moreover, the expression and the functions of FOLR1 in a tissue can be confirmed by administering folic acid conjugated to a radioisotope, $^{99m}$Tc-EC20 (etarfolatide), or the like to the body of a patient and observing the uptake of folic acid into the tissue.

The methods described above can be used for evaluating a pharmacodynamic effects before and after the administration when the therapeutic method of the invention and the method of the invention for enhancing a therapeutic effect are conducted or when the medicament of the invention is used, resulting in effective and efficient treatment.

A route of administration which is most effective for the treatment is preferably used as the route of administration of the human FOLR1-targeting drug and the antagonist for folic acid metabolism for using the therapeutic method, the method for enhancing a therapeutic effect or the medicament of the invention. Examples thereof include oral administration or parenteral administration such as intraoral, tracheobronchial, rectal, subcutaneous, intramuscular, intraperitoneal or intravenous administration, and intravenous administration is preferable.

When the medicament of the invention includes two or more preparations separately containing the human FOLR1-targeting drug and the antagonist for folic acid metabolism, the routes of administration thereof may be the same or different.

Examples of the dosage form include spray, capsules, tablets, powder, granules, syrup, emulsions, suppositories, injections, ointments, tapes or the like.

When the combined use agent of the invention includes two or more preparations separately containing the human FOLR1-targeting drug and the antagonist for folic acid metabolism, the dosage forms thereof may be the same or different.

The dose or the frequency of administration of the medicament of the invention varies with the therapeutic effect to be obtained, the administration method, the treatment period, the age, the body weight and the like, but the doses and the frequencies of the human FOLR1-targeting drug and the antagonist for folic acid metabolism for an adult are generally each 1 μg/kg to 1000 mg/kg per day.

The invention is explained more specifically below by Examples, but the invention is not limited to the Examples below.

## Examples

### [Example 1]

(1) Non-Small Cell Lung Cancer (NSCLC) Cell Lines cultured with pemetrexed

NSCLC cell lines such as NCI-H522 [American Type Culture Collection (hereinafter referred to as ATCC) No. CRL-5810], NCI-H838 (ATCC No. CRL-5844), NCI-H1437 (ATCC No. CRL-5872) and NCI-H2228 (ATCC No. CRL-5935) were cultured in a culture medium [RPMI1640 (GIBCO), 10% inactivated bovine serum (FCS, GIBCO)].

Next, cells were detached from culture flasks using TrypLE Express (GIBCO) and suspended in a folic acid-free medium [RPMI 1640 folate-free (GIBCO), 10% inactivated dialyzed FCS (dFCS, GIBCO)]. The cells were seeded to 25-$cm^2$ tissue culture (TC) flasks at $2.5\times10^5$ cells/4 mL.

After 24-hour incubation, 2 mL of a 3xlow folic acid medium (obtained by adding 1/33 amount of the culture medium to the folic acid-free medium) was added, and the culture was switched to a low folic acid condition having a final folic acid concentration of about 22.7 nmol/L. Furthermore, pemetrexed (Eli Lilly) was added to the culture at the final concentration of 0,10 or 1000 nmol/L. These cells were cultured for further 72 hours.

(2) Analysis of Expression Level of FOLR1 in NSCLC Cell Lines

After culturing each cell line under the above conditions, cells were detached using TrypLE Express, washed with phosphate buffur saline (PBS) and then suspended in FCM buffer (PBS containing 1 mmol/L EDTA, 0.05% sodium azide and 5% inactivated FCS).

Next, the cells were seeded in a 96-well plate at $1\times10^5$ cells per well and centrifuged at 1700 rpm for one minute. After removing the supernatant, an anti-human FOLR1 humanized antibody (HuRA15-7CTAcc antibody) (WO2014/087863) which was prepared at 10 $\mu$g/mL with FCM buffer was added to the cells and reacted for 30 minutes at 4°C.

After washing the cells, Anti-Human IgG-FITC (Jackson) which was diluted 100 times with the FCM buffer was added and reacted for 30 minutes at 4°C. After washing the cells again, the cells were suspended in the FCM buffer. The fluorescence intensities were analyzed with a flow cytometer (Beckman Coulter, Inc., FC500MPL) to determine the mean fluorescence intensity (MFI) of each cell.

Referring to the reports of WO2007/011041 and WO2011/108502, HuRA15-7CTAcc antibody was obtained by modifying the antibody heavy chain constant region of pKANTEX93, inserting genes encoding the amino acid sequences of SEQ ID NO: 8 and SEQ ID NO: 9 to the vector and expressing the antibody using Chinese hamster ovary cells (CHO/DG44) in which fucosyltransferase-8 (FUT8) gene had been knocked out. The expressed antibody was purified and then subjected to the experiment.

As a result, in NCI-H1437 and NCI-H2228, the expression levels of FOLR1 after the incubation with the final pemetrexed concentrations of 10 and 1000 nmol/L were about 1.5 times and 2 times higher than those after the incubation with the final pemetrexed concentration of 0 nmol/L [Fig. 1(A) and Fig. 1(B)], respectively. In NCI-H522 and NCI-H838, there was a tendency that the incubation with higher concentration of pemetrexed induced higher expression level of FOLR1 [Fig. 1(C) and Fig. 1 (D)].

These results showed that the expression level of FOLR1 increases when NSCLC cells are cultured with pemetrexed, which is an antagonist for folic acid metabolism. Thus, it was demonstrated that an antagonist for folic acid metabolism increases the expression level of FOLR1 in NSCLC cells.

[Example 2]

Culture of Ovarian Cancer Cell Lines with pemetrexed and Analysis of Expression Level of FOLR1

In a similar manner to that in Example 1, the expression level of FOLR1 in ovarian cancer cell lines was analyzed after incubation with pemetrexed. First, SKOV-3 [American Type Culture Collection (hereinafter referred to as ATCC) No. HTB-77], MCAS (JCRB cell No. JCRB0240), NIH:OVCAR-3 (ATCC No. HTB-161) and IGR-OV1 (National Cancer Institute), which are ovarian cancer-derived cell lines, were cultured in the culture medium. Then, detached cells were suspended in the folic acid-free medium, and seeded to 25-$cm^2$ TC flasks at $5\times10^5$ cells/4 mL.

In a similar manner to that in Example 1, the expression level of FOLR1 was analyzed after incubation with pemetrexed. As a result, in IGR-OV1, SKOV-3 and MCAS, there was a tendency that the incubation with higher concentration of pemetrexed induced higher expression level of FOLR1 [Fig. 2(A), Fig. 2(B) and Fig. 2(D)].

The expression levels of FOLR1 after the incubation with the final pemetrexed concentrations of 1000 nmol/L in the low folic acid medium were about 2.5 times higher in IGR-OV1 [Fig. 2(A)], about 2.5 times higher in SKOV-3 [Fig. 2(B)], and about 7 times higher in MCAS [Fig. 2(D)] than that of after the incubation in the culture medium (NC).

Moreover, in SKOV-3 and MCAS, the expression levels of FOLR1 were also increased after the incubation in the low folic acid medium without pemetrexed compared with that of after incubation in the culture medium [Fig. 2(B) and Fig. 2(D)].

On the other hand, there was a cell line in which the expression level of FOLR1 did not increase after the incubation in the low folic acid medium regardless of the final pemetrexed concentration as compared to that after the incubation in the culture medium [Fig. 2(C)].

These results demonstrated that there are ovarian cancer cell lines in which the expression level of FOLR1 increases when the cells are cultured in the presence of pemetrexed or cultured in a low folic acid medium. Furthermore, taken together with the results of Example 1, it was shown that the effect of pemetrexed on the increase of FOLR1 expression level is more remarkable in ovarian cancer cell lines than in NSCLC cell lines. It was also demonstrated that an antagonist for folic acid metabolism increases the expression level of FOLR1 not only in NSCLC but also in ovarian cancer.

[Example 3]

Evaluation of ADCC Activity in Pemetrexed-treated Cells

In a similar manner to that in Example 2, the ovarian cancer cell line MCAS was first cultured in the culture medium. Then, detached cells were suspended in the folic acid-free medium, and the cells were seeded to 175-cm$^2$ TC flasks at $25 \times 10^5$ cells/28 mL. After 24-hour incubation, 14 mL of the 3xlow folic acid medium was added, and the final folic acid concentration was adjusted at approximately 22.7 nmol/L. Then, pemetrexed was added to the culture at the final concentration of 0 or 20 nmol/L, and the cells were cultured for further 72 hours.

The cultured cells were detached from the culture flasks using 0.02% EDTA solution (Nacalai), and the cell counts and the viability were measured using Vi-CELL XR 2.01 (Beckman Coulter). As a result, the cell viability of the MCAS cells which incubated with 0 nmol/L or 20 nmol/L pemetrexed was 98.0% or 96.6%, respectively.

These cells were adjusted at $1 \times 10^4$ cells/50 μL using ADCC measurement medium [phenol red-free RPMI1640 (GIBCO), 5% inactivated dFCS (GIBCO)] as target cell solutions.

Next, heparin-treated peripheral blood of a healthy donor was loaded into a LeucoSep lymphocyte separation tube (Greiner) filled with Lymphoprep (Cosmo Bio), and centrifuged at 1000×g for 20 minutes at room temperature.

After the centrifugation, the plasma fraction was removed, and the peripheral blood mononuclear cell (PBMC) layer was collected. Then, the cells were washed with the ADCC measurement medium. The PBMCs were adjusted at $2.5 \times 10^5$ cells/50 μL using the ADCC measurement medium as an effector cell solution.

HuRA15-7CTAcc antibody and MORAb-003 (farletuzumab) were used as anti-human FOLR1 humanized antibodies. The antibody solutions were prepared at 0.1, 1, 10, 100 and 1000 ng/mL with the ADCC measurement medium, so that the final concentrations of the assay became 0.033, 0.33, 3.3, 33 and 333 ng/mL.

MORAb-003 was expressed using Chinese hamster ovary cells (CHO/DG44) which transfected a vector having genes encoding the sequences of the heavy chain and the light chain described in WO2005/080431. The expressed antibody was purified and then subjected to the experiment.

Fifty μL of each the target cell solution, the effector cell solution, and the antibody solution, which were prepared as described above, were mixed in a U-bottom 96-well plate, so that the total volume of each well was 150 μL. After mixing the wells, the cells were precipitated by centrifugation at 50×g for five minutes at room temperature. These cells were reacted for four hours at 37°C.

After mixing the wells, the cells were precipitated again by centrifugation. Then, 50 μL of the supernatant of each well was dispensed to a new 96-well plate. The lactate dehydrogenase (LDH) activities of the dispensed supernatant solutions were measured using CytoTox 96 Non-Radioactive Cytotoxicity Assay (Promega) according to the instruction.

The ADCC activity was calculated by the following equation after subtracting the measured LDH activity value of the well containing the ADCC measurement medium only from the measured LDH activity value of all wells. These values were determined as the corrected LDH activity value of each well.

$$\text{ADCC Activity (\%)} = 100 \times (\text{Exp} - \text{ET spo}) / (\text{T total} - \text{D} - \text{T spo})$$

Here, Exp is the corrected LDH activity value of a sample; ET spo is the corrected LDH activity value of the well in which the effector cell solution and the target cell solution were mixed; T total is the corrected LDH activity value of the well in which the target cells and the Lysis Solution attached to CytoTox 96 Non-Radioactive Cytotoxicity Assay were mixed; D is the corrected LDH activity value of the Lysis Solution; and T spo is the corrected LDH activity value of the well containing the target cells only.

As a result of the ADCC activity study using HuRA15-7CTAcc antibody using PBMCs of two healthy donors, cytotoxicity was observed in association with the antibody concentration [Fig. 3(A) and Fig. 3(B)]. The ADCC activity of HuRA15-7CTAcc antibody against MCAS cells cultured with pemetrexed was significantly higher than the ADCC activity of HuRA15-7CTAcc antibody against MCAS cells cultured without pemetrexed, at all the antibody concentrations in the results of the assay using the healthy donor 1-derived PBMCs [Fig. 3(A)], and at the antibody concentrations of 0.033 and 0.33 ng/mL in the results of the assay using the healthy donor 2-derived PBMCs [Fig. 3(B)].

In the ADCC activity test using MORAb-003 antibody, which is an existing anti-human FOLR1 antibody, the ADCC activity of MORAb-003 antibody against MCAS cells cultured with pemetrexed was significantly higher [Fig. 3(B)] than the ADCC activity of MORAb-003 antibody against MCAS cells cultured without pemetrexed at the antibody concentrations of 0.33, 3.3, 33 and 333 ng/mL in the results of the assay using the healthy donor 2-derived PBMCs [Fig. 3(B)].

From the above results, it was shown that the ADCC activity of an anti-human FOLR1 antibody is enhanced when an ovarian cancer cell line cultured with pemetrexed is the targets. Therefore, it was demonstrated that the ADCC activity of an anti-human FOLR1 antibody, which is a human FOLR1-targeting drug, is enhanced against a cancer which FOLR1 expression level is increased by an antagonist for folic acid metabolism.

[Example 4]

Combination Use of Pemetrexed and Anti-Human FOLR1 Antibody in SCID Mouse subcutaneously transplanted Ovarian Cancer Cell Line MCAS

The ovarian cancer cell line MCAS at about 2.5×10$^6$ cells suspended in PBS was subcutaneously transplanted in the ventral part of female SCID mice. Eleven days after the transplantation, the body weights and the tumor sizes were measured. The tumor volumes were calculated by the following equation. Then, the mice were divided into five groups (N=5) by E Workbook software (ID Business Solutions Ltd) in order that the mean of tumor volume in each group was comparable.

$$[\text{Tumor Volume (mm}^3)] = [\text{Length of Tumor (mm)}] \times [\text{Breadth of Tumor (mm)}]^2 \times 0.5$$

The test was conducted in five groups: a vehicle administration group, an anti-human FOLR1 antibody (HuRA15-7CTAcc antibody) single administration group, a pemetrexed single administration group, a simultaneous combination group, and a sequential combination group. Physiological saline was administered to the vehicle group. To the anti-human FOLR1 antibody administration group, the antibody solution diluted by physiological saline was administered to the tail vein at 1 mg/kg twice a week for two weeks. To the pemetrexed administration group, pemetrexed diluted by physiological saline was administered intraperitoneally at 100 mg/kg/day for five days after starting the first administration. To the simultaneous combination group, both drugs were administered in similar manners to those in the single agent groups. To the sequential combination group, pemetrexed was administered intraperitoneally for the first five days, and from three days after the final administration of pemetrexed, 1 mg/kg of the anti-human FOLR1 antibody was administered twice a week for a week.

During the study, the body weight and the tumor volume were measured twice a week. The tumor volume on each measurement day was calculated by the above equation. The tumor growth rate (hereinafter referred to as V/V0) was calculated by dividing the tumor volume V on the measurement day by the tumor volume V0 on the start day of the test (day 0). T/C was calculated by dividing the average of V/V0 of each group (T) by the average of the vehicle administration group (C).

As a result, the tumor volumes, the tumor growth rates, and T/C of both the anti-human FOLR1 antibody and pemetrexed simultaneous combination group and the sequential combination group were smaller than those of both the anti-human FOLR1 antibody single administration group and the pemetrexed single administration group [Fig. 4(A), Fig. 4(B) and Fig. 4(C)].

That is, it was suggested that the anti-tumor activity of the combination of the anti-human FOLR1 antibody and pemetrexed is enhanced as compared to the anti-tumor activity of the single use of the anti-human FOLR1 antibody or pemetrexed. In addition, the body weight did not decrease even when the anti-human FOLR1 antibody was used in combination with pemetrexed [Fig. 4(D)].

Thus, it was demonstrated that simultaneous or sequential administration of an antagonist for folic acid metabolism enhances the anti-tumor activity of a human FOLR1-targeting drug and more effectively suppresses the tumor volume and the tumor growth rate.

[Example 5]

Culture of Endometrial Cancer Cell Lines with pemetrexed and Analysis of Expression Level of FOLR1

Endometrial cancer-derived cell line MES-SA (ATCC No. CRL-1976) and HEC-1-A (ATCC No. HTB-112) were cultured in McCoy's 5A medium (ATCC) containing 10% FCS (GIBCO), and HEC-1-B (ATCC No. HTB-113) was cultured in Eagle's Minimum Essential Medium (ATCC) containing 10% FCS (GIBCO). Then, detached cells were suspended in the folic acid-free medium, and the cells were seeded to 25-cm$^2$ TC flasks at 2.5×10$^5$ cells/4 mL, as described in Example 1.

After 24-hour incubation, 2 mL of a 3×low folic acid medium (obtained by adding 1/33 amount of the culture medium to the folic acid-free medium) was added, and the culture was switched to a low folic acid condition medium having a final folic acid concentration of approximately 22.7 nmol/L. Furthermore, pemetrexed (Eli Lilly) was added to the culture at the final concentration of 0,10 or 1000 nmol/L. These cells were cultured for further 72 hours.

Next, the expression level of FOLR1 was analyzed by a similar method to that in Example 1 except for using 10 times diluted Anti-Human IgG-PE (BD) by the FCM buffer as the labelled antibody instead of 100 times diluted Anti-Human IgG-FITC (Jackson) by the FCM buffer. Also, a flow cytometer manufactured by Becton, Dickinson and Company (FACSVerse) was used instead of the flow cytometer manufactured by Beckman Coulter, Inc. (FC500MPL).

As a result, there was a tendency that higher pemetrexed treatment induced higher expression level of FOLR1 in MES-SA, HEC-1-A and HEC-1-B cells [Fig. 5(A), Fig. 5(B) and Fig. 5(C)]. The expression levels of FOLR1 after the culture with the final pemetrexed concentrations of 1000 nmol/L in the low folic acid medium were approximately 3.5 times in MES-SA [Fig. 5(A)], approximately 3 times in HEC-1-A [Fig. 5(B)], and approximately 2 times in HEC-1-B [Fig. 5(C)] as high as those after the culture in the culture medium (NC).

According to these results, it was shown that the expression level of FOLR1 increases when endometrial cancer cells are cultured in the presence of pemetrexed. Therefore, it was demonstrated that an antagonist for folic acid metabolism increases the expression level of FOLR1 not only in NSCLC and ovarian cancer but also in endometrial cancer.

[Example 6]

Evaluation of ADCC Activity in Pemetrexed-Treated Cells (NSCLC Cell Line)

The ADCC activity against the NSCLC cell line NCI-H2228 was evaluated by a similar method to that in Example 3 except for using pemetrexed at the final concentration of 0, 20 or 1000 nmol/L in the culture. Also, the cell counts and the viability were measured using Countess (Life Technologies) instead of Vi-CELL XR 2.01.

The ADCC activity of HuRA15-7CTAcc antibody was evaluated using PBMCs of a healthy donor. As a result, cytotoxicity was observed in association with the antibody concentration. Moreover, the ADCC activity of HuRA15-7CTAcc antibody against NCI-H2228 cells cultured with 1000 nmol/L pemetrexed was significantly higher than the ADCC activity of HuRA15-7CTAcc antibody against NCI-H2228 cells cultured without pemetrexed at all the antibody concentrations [Fig. 6(A)].

In the ADCC activity test using MORAb-003 antibody, which is an existing anti-human FOLR1 antibody, the ADCC activity of MORAb-003 antibody against NCI-H2228 cells cultured with 1000 nmol/L pemetrexed was significantly higher than the ADCC activity of MORAb-003 antibody against NCI-H2228 cells cultured without pemetrexed at the antibody concentrations of 0.0033, 3.3, 33 and 333 ng/mL [Fig. 6(B)].

From the above results, it was shown that the ADCC activity of an anti-human FOLR1 antibody is enhanced when a NSCLC cell line cultured with pemetrexed is the target. Therefore, it was demonstrated that the ADCC activity of an anti-human FOLR1 antibody, which is a human FOLR1-targeting drug, is enhanced against a cancer which FOLR1 expression level is increased by an antagonist for folic acid metabolism.

The invention has been explained in detail using specific embodiments, but it is obvious to one skilled in the art that various changes and modifications are possible without departing from the purpose and the scope of the invention. This application is based on a US provisional application filed on June 6, 2014 (No. 62/008,726), which is hereby incorporated by reference in its entirety.

Sequence Listing Free Text

SEQ ID NO: 1: Amino acid sequence of FOLR1
SEQ ID NO: 2: Amino acid sequence of RA15-7VHCDR1_AA
SEQ ID NO: 3: Amino acid sequence of RA15-7VHCDR2_AA
SEQ ID NO: 4: Amino acid sequence of RA15-7VHCDR3_AA
SEQ ID NO: 5: Amino acid sequence of RA15-7VLCDR1_AA
SEQ ID NO: 6: Amino acid sequence of RA15-7VLCDR2_AA
SEQ ID NO: 7: Amino acid sequence of RA15-7VLCDR3_AA
SEQ ID NO: 8: Amino acid sequence of nonS_HuRA15-7CTVH_AA
SEQ ID NO: 9: Amino acid sequence of nonS_HuRA15-7LV3_AA
SEQ ID NO: 10: Amino acid sequence of nonS_RA15-7VH_AA
SEQ ID NO: 11: Amino acid sequence of nonS_RA15-7VL_AA
SEQ ID NO: 12: Nucleotide sequence of FOLR1 DNA
SEQ ID NO: 13: Amino acid sequence of HuRA15-7CTAccVHCDR3_AA

## Claims

1. A method for treating a cancer by using a human FOLR1-targeting drug, wherein an expression of folate receptor $\alpha$ (hereinafter abbreviated to FOLR1) is increased by using an antagonist for folic acid metabolism.

2. The method according to claim 1, wherein the antagonist for folic acid metabolism is administered simultaneously or sequentially.

3. The method according to claim 1 or 2, wherein the cancer is a cancer which expresses FOLR1.

4. The method according to any one of claims 1 to 3, wherein the human FOLR1-targeting drug is any one selected from a FOLR1 inhibitor, an anti-human FOLR1 antibody and an anti-human FOLR1 antibody fragment.

5. The method according to claim 4, wherein the FOLR1 inhibitor is a folic acid derivative.

6. The method according to claim 4, wherein the anti-human FOLR1 antibody and the antibody fragment are an antibody and a fragment of the antibody which bind to an epitope contained in the amino acid sequence of from position 55 to position 62 in the amino acid sequence of SEQ ID NO: 1.

7. A method for enhancing a therapeutic effect of a human FOLR1-targeting drug by increasing an expression of FOLR1 in a cancer cell by using an antagonist for folic acid metabolism.

8. The method according to claim 7, wherein the antagonist for folic acid metabolism is administered simultaneously or sequentially.

9. The method according to claim 7 or 8, wherein the cancer is a cancer which expresses FOLR1.

10. The method according to any one of claims 7 to 9, wherein the human FOLR1-targeting drug is any one selected from a FOLR1 inhibitor, an anti-human FOLR1 antibody and an anti-human FOLR1 antibody fragment.

11. The method according to claim 10, wherein the FOLR1 inhibitor is a folic acid derivative.

12. The method according to claim 10, wherein the anti-human FOLR1 antibody and the antibody fragment are an antibody and a fragment of the antibody which bind to an epitope contained in the amino acid sequence of from position 55 to position 62 in the amino acid sequence of SEQ ID NO: 1.

13. A medicament for a cancer containing a human FOLR1-targeting drug for increasing an expression of FOLR1 in a cancer cell by administering an antagonist for folic acid metabolism.

14. The medicament according to claim 13, wherein the antagonist for folic acid metabolism is administered simultaneously or sequentially.

15. The medicament according to claim 13 or 14, wherein the cancer is a cancer which expresses FOLR1.

16. The medicament according to any one of claims 13 to 15, wherein the human FOLR1-targeting drug is any one selected from a FOLR1 inhibitor, an anti-human FOLR1 antibody and an anti-human FOLR1 antibody fragment.

17. The medicament according to claim 16, wherein the FOLR1 inhibitor is a folic acid derivative.

**18.** The medicament according to claim 16, wherein the anti-human FOLR1 antibody and the antibody fragment are an antibody and a fragment of the antibody which bind to an epitope contained in the amino acid sequence of from position 55 to position 62 in the amino acid sequence of SEQ ID NO: 1.

[Fig. 1]

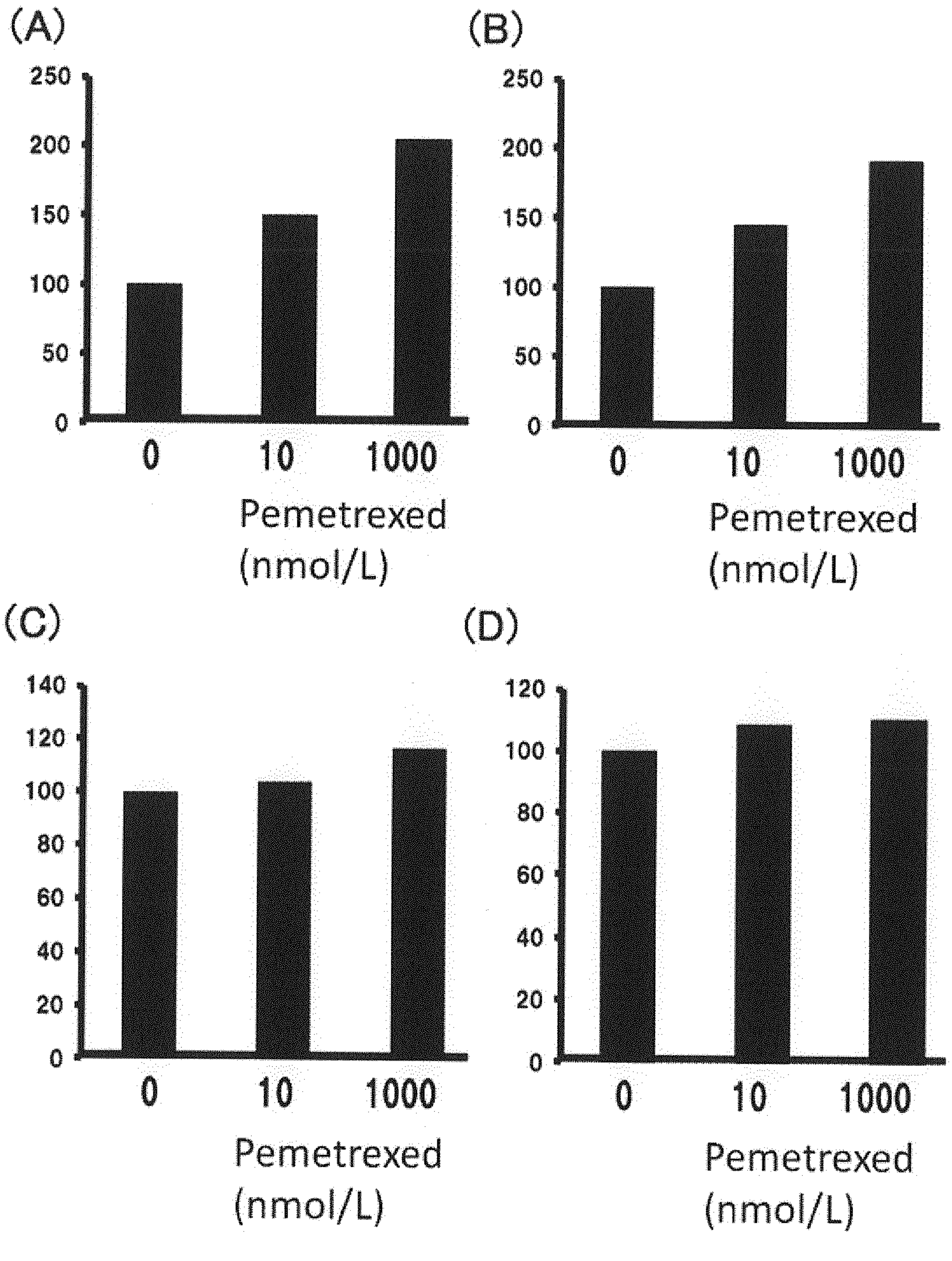
(A)
250
200
150
100
50
0
0
10
1000
Pemetrexed (nmol/L)
(B)
250
200
150
100
50
0
0
10
1000
Pemetrexed (nmol/L)
(C)
140
120
100
80
60
40
20
0
0
10
1000
Pemetrexed (nmol/L)
(D)
120
100
80
60
40
20
0
0
10
1000
Pemetrexed (nmol/L)

[Fig. 2]

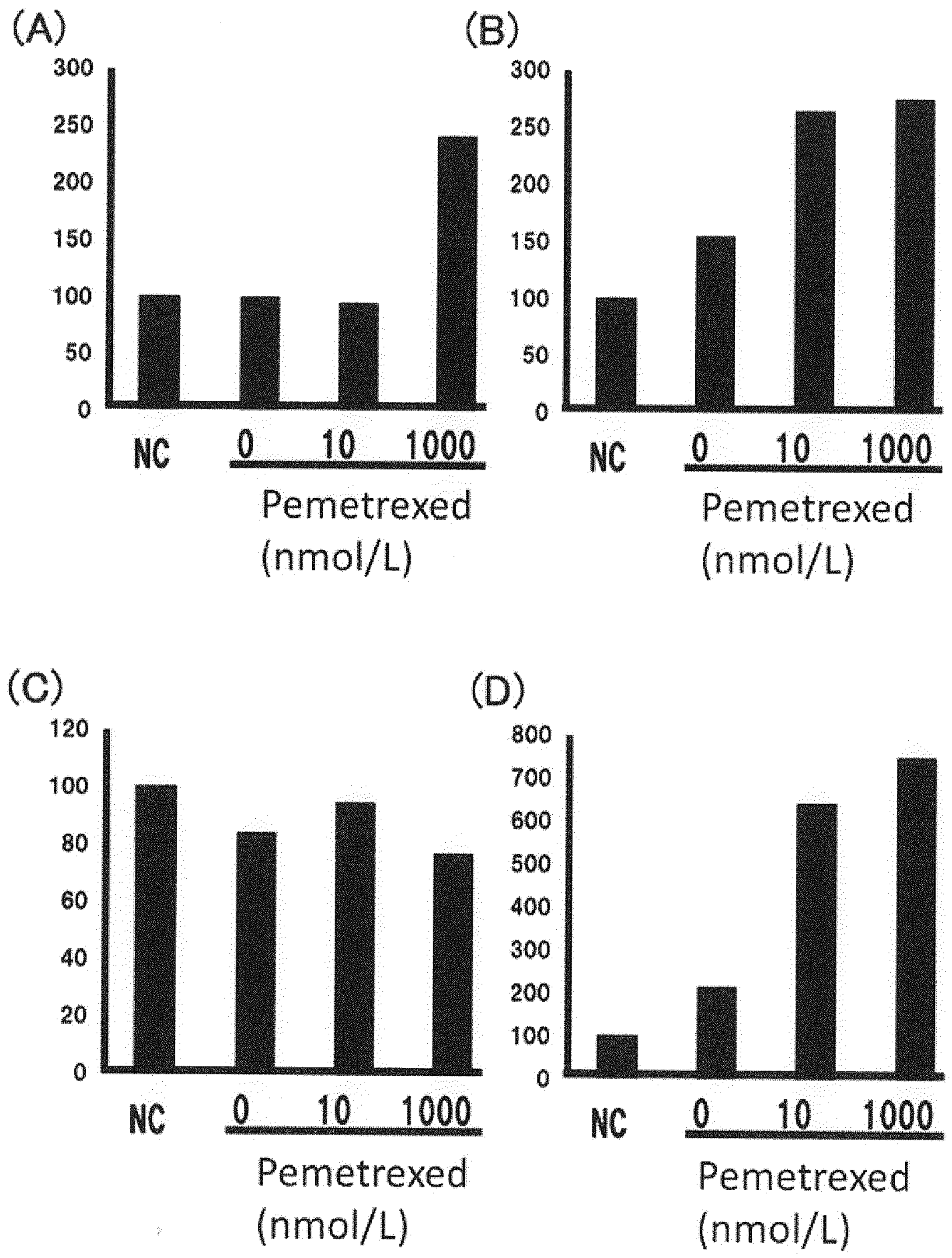
(A)
300
250
200
150
100
50
0
NC
0
10
1000
Pemetrexed (nmol/L)
(B)
300
250
200
150
100
50
0
NC
0
10
1000
Pemetrexed (nmol/L)
(C)
120
100
80
60
40
20
0
NC
0
10
1000
Pemetrexed (nmol/L)
(D)
800
700
600
500
400
300
200
100
0
NC
0
10
1000
Pemetrexed (nmol/L)

[Fig. 3]

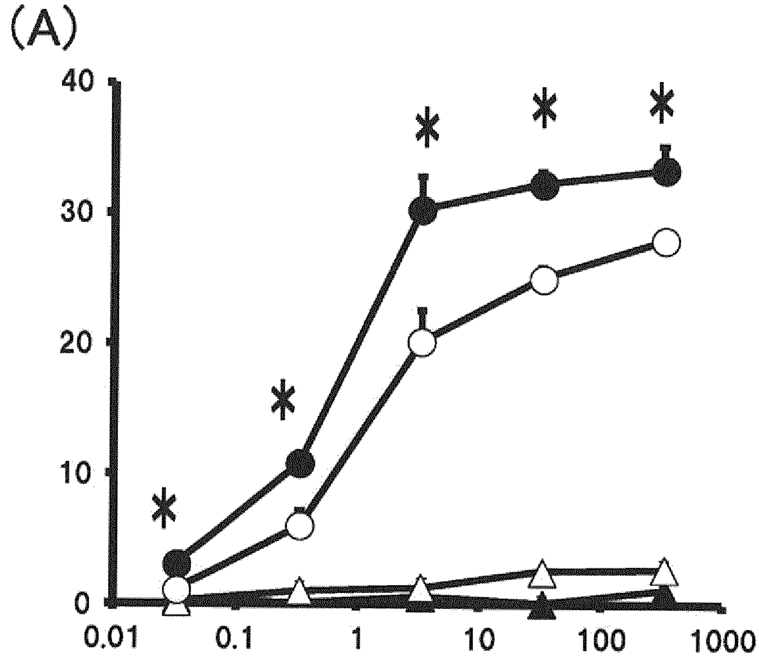
(A)
40
30
20
10
0
0.01
0.1
1
10
100
1000
*
*
*
*
*
*

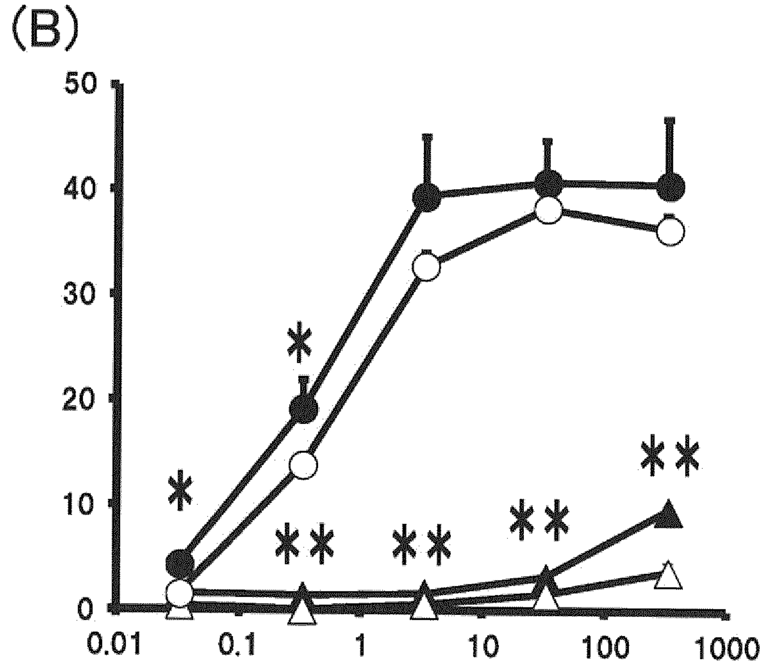
(B)
50
40
30
20
10
0
0.01
0.1
1
10
100
1000
*
*
**
**
**
**

[Fig. 4]

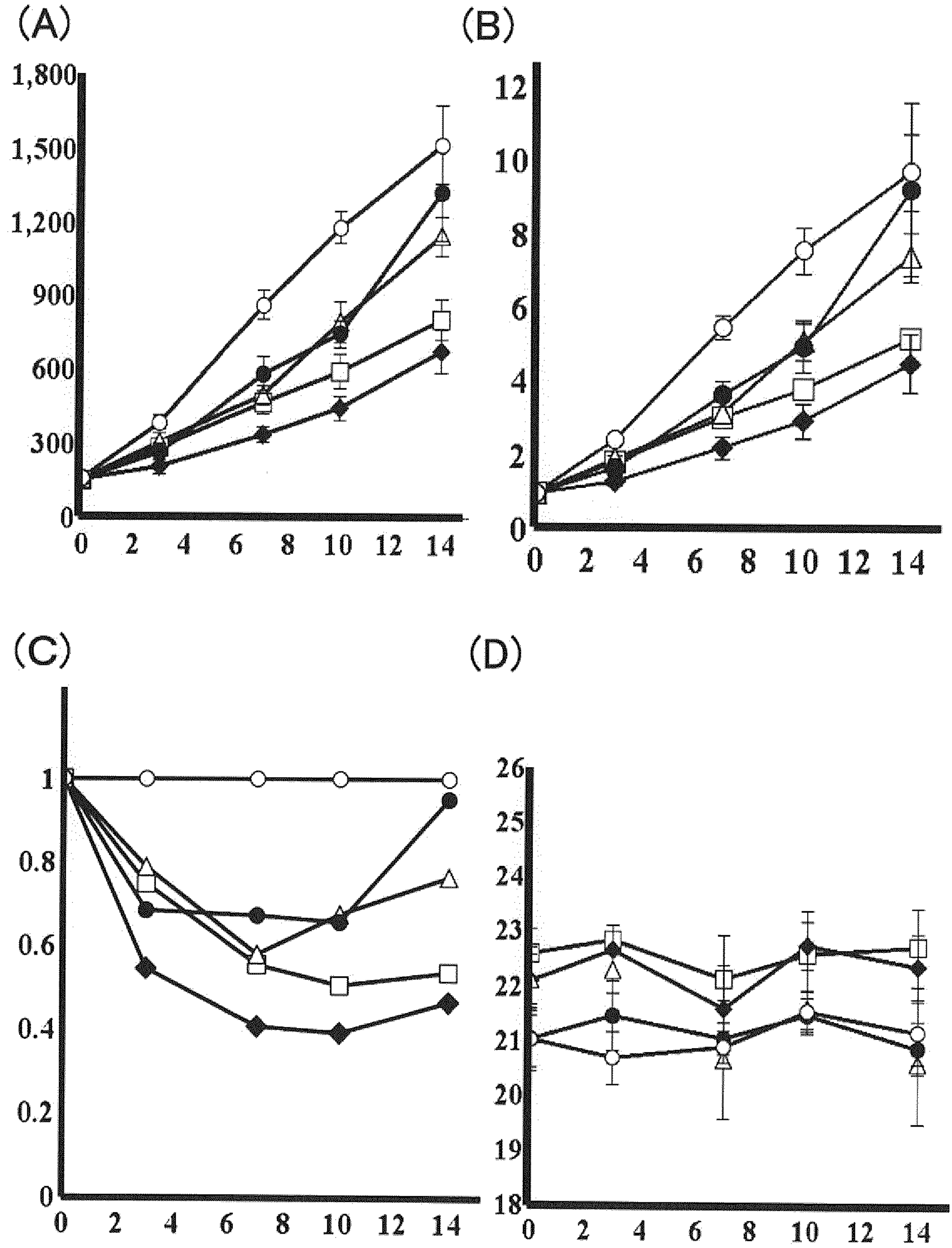
(A)
1,800
1,500
1,200
900
600
300
0
0 2 4 6 8 10 12 14
(B)
12
10
8
6
4
2
0
0 2 4 6 8 10 12 14
(C)
1
0.8
0.6
0.4
0.2
0
0 2 4 6 8 10 12 14
(D)
26
25
24
23
22
21
20
19
18
0 2 4 6 8 10 12 14

[Fig. 5]

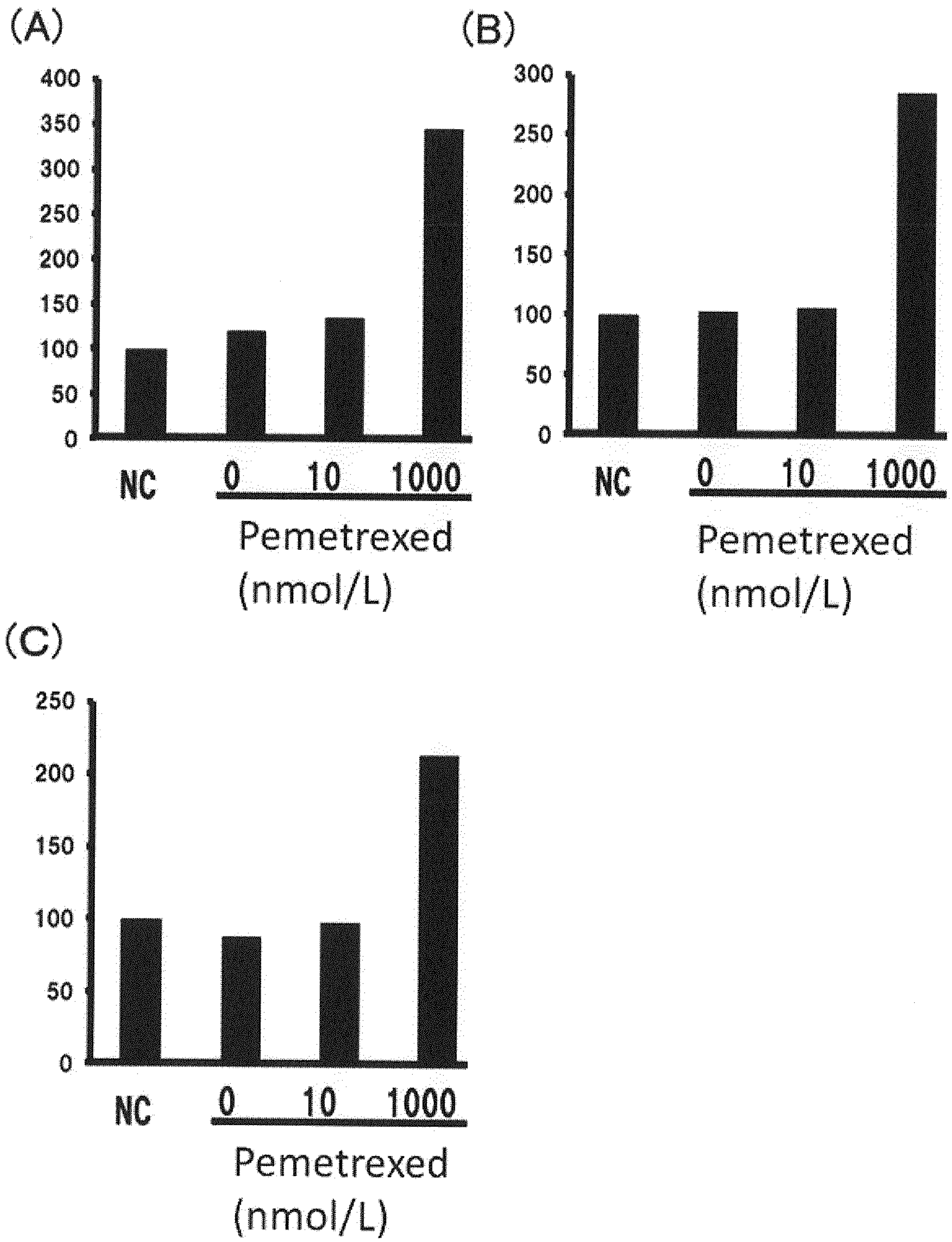
(A)
400
350
300
250
200
150
100
50
0
NC
0
10
1000
Pemetrexed (nmol/L)
(B)
300
250
200
150
100
50
0
NC
0
10
1000
Pemetrexed (nmol/L)
(C)
250
200
150
100
50
0
NC
0
10
1000
Pemetrexed (nmol/L)

[Fig. 6]

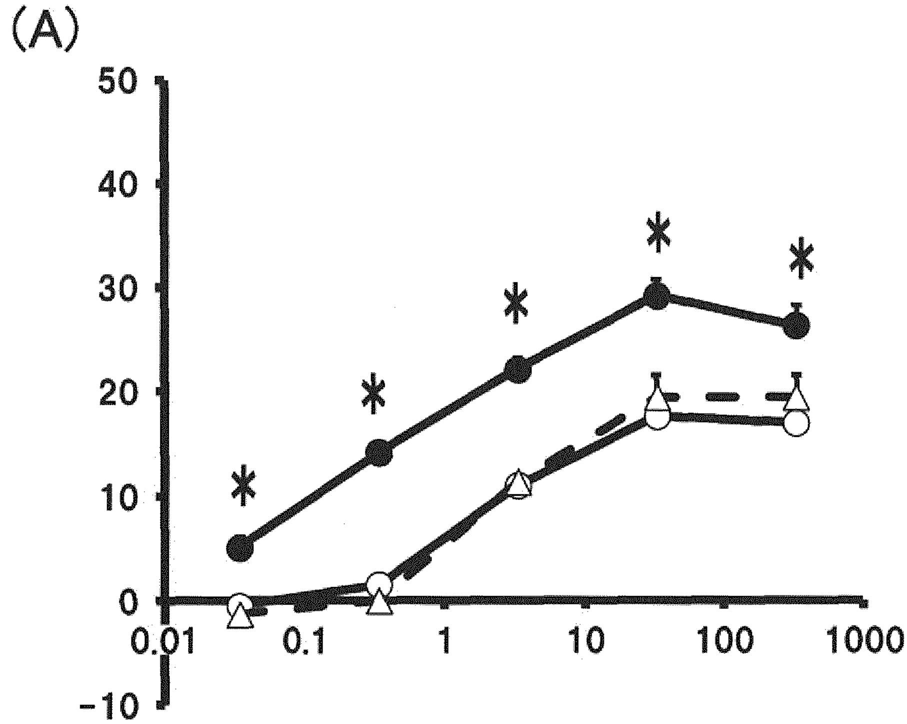
(A)
50
40
30
20
10
0
-10
0.01
0.1
1
10
100
1000
*

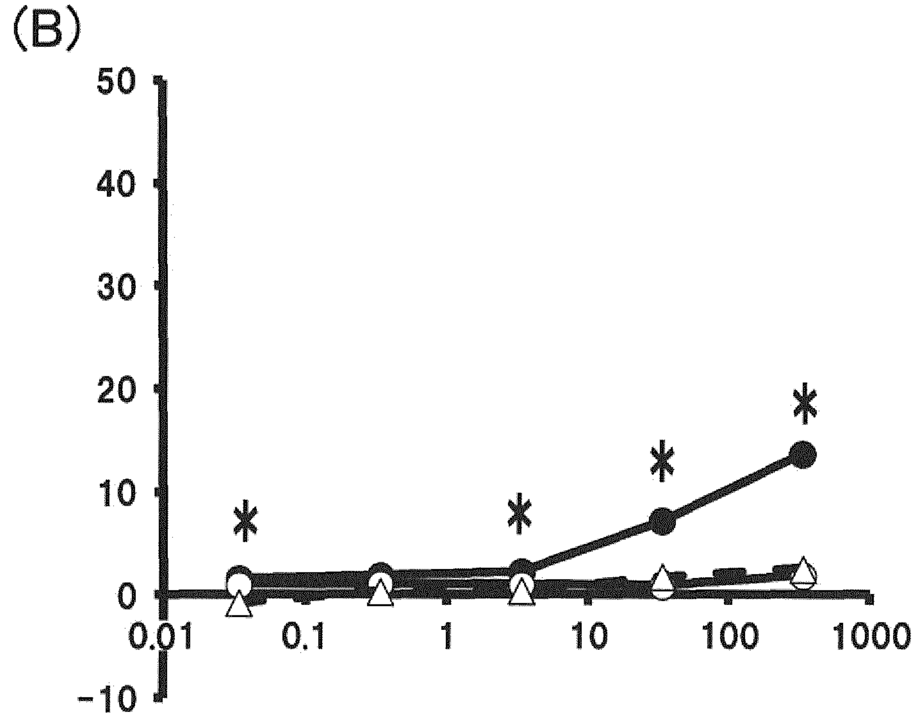
(B)
50
40
30
20
10
0
-10
0.01
0.1
1
10
100
1000
*

**INTERNATIONAL SEARCH REPORT**

International application No. PCT/JP2015/066328

A. CLASSIFICATION OF SUBJECT MATTER
*A61K45/00*(2006.01)i, *A61K31/519*(2006.01)i, *A61K39/395*(2006.01)i, *A61P35/00*(2006.01)i

According to International Patent Classification (IPC) or to both national classification and IPC

B. FIELDS SEARCHED

Minimum documentation searched (classification system followed by classification symbols)
A61K45/00, A61K31/519, A61K39/395, A61P35/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2015 |
| Kokai Jitsuyo Shinan Koho | 1971-2015 | Toroku Jitsuyo Shinan Koho | 1994-2015 |

Electronic data base consulted during the international search (name of data base and, where practicable, search terms used)
JSTPlus/JMEDPlus/JST7580(JDreamIII), CAplus/MEDLINE/EMBASE/BIOSIS(STN)

C. DOCUMENTS CONSIDERED TO BE RELEVANT

| Category* | Citation of document, with indication, where appropriate, of the relevant passages | Relevant to claim No. |
|---|---|---|
| X<br>Y | THE JOURNAL OF NUCLEAR MEDICINE, 2006, Vol.47, No.12, p.2057-2064 | 1-5,7-11, 13-17<br>4,6,10,12, 16,18 |
| Y | Expert Opinion on Drug Delivery, 2012, Vol.9, No.8, p.901-908 | 4,6,10,12, 16,18 |
| Y | Cancer Chemother Pharmacol, 2012, Vol.70, p.113-120 | 4,6,10,12, 16,18 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

Date of the actual completion of the international search
18 August 2015 (18.08.15)

Date of mailing of the international search report
25 August 2015 (25.08.15)

Name and mailing address of the ISA/
Japan Patent Office
3-4-3,Kasumigaseki,Chiyoda-ku,
Tokyo 100-8915,Japan

Authorized officer

Telephone No.

Form PCT/ISA/210 (second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0197435 A
- US 5952484 A
- WO 2005035586 A
- WO 200231140 A
- WO 0061739 A
- US 20070148165 A
- US 6737056 B
- US 7297775 B
- US 7317091 B
- WO 2014087863 A
- WO 2007011041 A
- WO 2011108502 A
- WO 2005080431 A
- US 62008726 B


### Non-patent literature cited in the description


- *Int J Cancer,* 2006, vol. 119 (2), 243-50
- *Anal Biochem,* 2005, vol. 338 (2), 284-93
- *J Thorac Oncol,* 2012, vol. 7 (5), 833-840
- **J THORAC.** *Cardiovasc Surg,* 2001, vol. 121 (2), 225-33
- *Int J Cancer,* 1997, vol. 74 (2), 193-8
- *Int J Cancer,* 1998, vol. 79 (2), 121-6
- *PLoS One,* 2009, vol. 4 (7), e6292
- News Release. Eisai Co., Ltd, 11 January 2013
- News Release. Morphotek Inc, 02 December 2011
- **MALTZMAN, J. D. et al.** *World Conf Lung Cancer,* 2013
- *J Clin Oncol,* 2013, vol. 31, 4400-4406
- PRESS RELEASES. Endocyte, Inc, 21 March 2014
- *Nat Rev Cancer,* 2011, vol. 11 (10), 719-25
- *Cancer Metastasis Rev,* 2007, vol. 26, 153-181
- *Drug Metab Dispos.,* June 1997, vol. 25 (6), 693-700
- *CHEMICAL ABSTRACTS,* 357166-29-1
- *Curr Opin Investig Drugs,* December 2010, vol. 11 (12), 1424-33
- *Cancer Immun.,* 09 March 2007, vol. 7, 6
- *Cancer Immunol. Immunother.,* 1993, vol. 36, 373
- *Clark M, Chemical Immunology,* 1997, vol. 65, 88
- **GORTER A et al.** *Immunol. Today,* 1999, vol. 20, 576
- *Immunol Today,* December 1999, vol. 20 (12), 576-82